# EUROPEAN PATENT APPLICATION

(11) **EP 4 043 481 A1**
(43) Date of publication of application: **17.08.2022**
(21) Application number: 21305193.1
(22) Date of filing: 15.02.2021
(51) Int. Cl.: C07K 14/195, C07K 14/765, C07K 19/00, A61K 38/00

(54) **COMPOUNDS AND METHODS FOR EXTENDING HALF LIFE OF BIOMOLECULES**

(71) Applicant: AFFILOGIC, 44200 Nantes (FR)
(72) Inventor: The designation of the inventor has not yet been filed
(74) Representative: Flesselles, Bruno F.G.

(57) **Abstract**

The invention relates to variants of OB-fold proteins, in particular of the Sac7d family that bind to human and mouse serum albumin and that can be used to increase serum half-life of elements associated therewith.

## Description

The invention is in the field of extending plasma half-life of proteins and relates in particular to the development of new binders to albumin usable for such purpose.

### Introduction

Translating laboratory experiments into *in vivo* environments to assess the safety and efficacy of a molecule requires that additional parameters are taken into account. In the case of a product that is exposed to the systemic circulation, the most important change is the impact of the residency of the product in the vascular flow. Managing the residence time in the bloodstream is necessary and can be provided for by using albumin, a most abundant circulating protein, as a temporary carrier. This can be achieved by transiently linking the compound of interest to serum albumin by way of a specific ligand that is fused or conjugated to said compound.

### SUMMARY OF THE INVENTION

The invention relates a polypeptide comprising a variant of the Sac7d protein or of a protein of the Sac7d family, in particular Aho7c that binds to albumin and its use in particular for extending the half-life of a molecule (including a peptide or polypeptide) bound thereto *in vivo.*

This variant binds both to human and murine serum albumin, which makes it valuable to speed up clinical development of products and translation from animal to human. Indeed, most of binders to serum albumin don't cross-react between mice and human. Furthermore, the affinity is important, thus guaranteeing a strong binding (and low turnover) of the variants to serum albumin (and hence increase of serum half-life of the biomolecule associated with the variants). The variants also bind to an epitope of serum albumin that doesn't hamper binding of serum albumin to FcR and thus recycling of the complex serum albumin / variant associated with the biomolecule, which proves interesting for increase of serum half-life of the variant associated with the biomolecule. The variant, being a small protein, doesn't diminish the own biological activity of the biomolecule to which it is associated.

In particular, in certain embodiments said variant is based on the Sac7d protein, and comprises SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64 or SEQ ID NO: 65. In certain embodiments, said variant is based on the Ahoc protein, and comprises SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85 or SEQ ID NO: 86. In case of discrepancy between the specification and the sequence listing, the sequences mentioned in the specification are preponderant.

In particular, the variant comprises SEQ ID NO: 38 or amino acids 1-54 of SEQ ID NO: 38.

In particular, the variant comprises a sequence selected from SEQ ID NO: 48, SEQ ID NO: 51, SEQ ID NO: 55, SEQ ID NO: 58 SEQ ID NO: 62, SEQ ID NO: 65, and amino acids 1-57 of these sequences.

In particular, the variant comprises a sequence selected from SEQ ID NO: 69, SEQ ID NO: 72, SEQ ID NO: 76, SEQ ID NO: 79 SEQ ID NO: 83, SEQ ID NO: 86, and amino acids 1-55 of these sequences.

The invention also relates to nucleic acid coding for the variants, polypeptides including the variants and an active substance, methods of production, and of use of the variants alone or combined with active substances.

In particular, the invention relates to
- A polypeptide herein disclosed which consists in the variant of a member of the Sac7d family binding to human and mouse serum albumin.
- A polypeptide herein disclosed, wherein the variant of a member of the Sac7d family binding to human and mouse serum albumin is conjugated to an organic molecule.
- A polypeptide herein disclosed, wherein the variant of a member of the Sac7d family binding to human and mouse serum albumin is conjugated to another polypeptide.
- A polypeptide herein disclosed, wherein the other polypeptide is another variant of a protein of the Sac7d family.
- A nucleic acid molecule coding for a polypeptide herein disclosed.
- An expression vector comprising a nucleic acid molecule herein disclosed.
- A host cell comprising a nucleic acid molecule herein disclosed, or the expression vector herein disclosed.
- A pharmaceutical composition comprising a polypeptide herein disclosed, a nucleic acid herein disclosed, an expression vector herein disclosed, or a host cell herein disclosed, and a pharmaceutically acceptable carrier
- A Method for producing a polypeptide herein disclosed, comprising
   (a)Culturing a cell culture wherein the cells have been transformed by an expression vector herein disclosed,
      And
   (b) Recovering the polypeptide.
- A polypeptide herein disclosed as a medicament.
- A polypeptide herein disclosed for use for the treatment of prevention of a disease, wherein the variant is associated with an substance active for treatment or prevention of the disease.

### DETAILED DESCRIPTION OF THE INVENTION

It is to be noted that the sequences indicated above may comprise a methionine at its N-terminal, but that it is also possible to perform the invention when such methionine listed at the N-terminal end of each sequence is not included. Similarly, the amino acids located at the end-terminus of the proteins may be omitted. In particular the amino acids located after residue L58 of Sac7d (residues located after L60 of SEQ ID NO: 16) may be omitted. Consequently, the variant may comprise amino acids 1-57, 1-58, 1-59, 1-60, 1-61, 1-62, or 1-63 of any of SEQ ID NO: 45 to SEQ ID NO: 65.

When based on the Aho7c scaffold, the variant may comprise amino acids 1-55, 1-56, 1-57, or 1-58 of any of SEQ ID NO: 66 to SEQ ID NO: 86.

Such variant is able to be fused to a biomolecule and/or conjugated to a small chemical entity and extend plasma half-life of the molecule, thus increasing efficacy or bioavailability, or allowing use of a lesser amount. Typical molecules that require such half-life extension are small chemical molecules (e.g. cytotoxic agents against cancer such as auristatin or doxorubicin), peptides (insulin analogs such as GLP-1), small protein scaffolds such as Sac7d from *Sulfolobus acidocaldarius* (and analogs such as Aho7c, or Sso7d) derivatives, antibody mimetics such as affibodies, affilins, affimers, alphabodies, anticalins, avimers, DARPins, fynomers, Kunits domain peptides, monobodies, Z domain of Protein A, Gamma B crystalline, ubiquitin, cystatin, , lipocalin, A domain of a membrane receptor, ankyrin repeat motive, SH3 domain of Fyn, Kunits domain of protease inhibitors, the 10th type III domain of fibronectin, 3- or 4-helix bundle proteins, an armadillo repeat domain, a leucine-rich repeat domain, a PDZ domain, a SUMO or SUMO-like domain, an immunoglobulin-like domain, phosphotyrosine-binding domain, pleckstrin homology domain, src homology 2 domain or synthetic peptide ligands, antibody fragments (including Fabs, VHH, ScFv), enzymes, therapeutic nucleic acids such as Antisense Oligonucleotides, siRNAs, shRNAs.

This is of particular interest for biomolecules aiming at neutralizing circulating molecules such as cytokines (e.g. IL-17, TNF-alpha...), growth factors, hormones, antimicrobial peptides, complement factors, coagulation factors; also for biomolecules that interact with circulating cells such as immune cells (T-Lymphocytes, Macrophages, Antigen Presenting Cells,...) for neutralization, recruitment or activation ; for biomolecules that require diffusion into tissues and organs and therefore benefit from a prolonged exposure in the vasculature to enhance the extravasation and/or tissue uptake, for example in the case of brain uptake, liver, lung or bone targeting ; similarly, higher diffusion into solid tumors is favored by a long residence time in the circulation leading to an enhanced antitumor activity (e.g. when neutralizing PD-L1 or EGF-R) ; for molecules with an antimicrobial or antiviral activity to cure sepsis of more generally infections.

Depending on the biomolecule to which the variant is fused, the fusion product can be used for treating cancer, metabolic diseases (such as diabetes), neurologic disease, inflammatory and autoimmune diseases (such as rheumatoid arthritis, psoriasis, lupus or multiple sclerosis), or enzyme deficiency in particular in the brain.

The biomolecule (polypeptide or organic molecule) is preferably biologically active. This indicates that this biomolecule interacts with molecules present in the subject's body (receptors, ligands, antigens and the like), and advantageously provides a beneficial effect to the subject (cure, relief, decrease of symptoms, decrease of markers associated with a disease to be treated (for instance decrease in viral load in case of viral infection). Indeed, an active substance is a substance or mixture of substances intended to be used in the manufacture of a medicinal product (or diagnostic product) and that, when used in its production, becomes an active ingredient of that product intended to exert a pharmacological, immunological or metabolic action with a view to restoring, correcting or modifying physiological functions or to make a medical diagnosis.

It is to be noted that the specification discloses variants of Sac7d, but the teachings are applicable to the other proteins of the Sac7d family. The teachings are also applicable to other OB-fold domains as disclosed in WO2007139397. The invention is also applicable to SH3 domains, a small protein domain of about 60 amino acid residues, initially, described as a conserved sequence in the viral adaptor protein v-Crk and described under PF00018 in the PFAM database. The SH3 domain has a characteristic beta-barrel fold that consists of five or six β-strands arranged as two tightly packed anti-parallel β sheets. The linker regions may contain short helices. It is to be noted that OB-fold and SH3 domains share homology and that, in view of the knowledge of the sequence and structure of these domains, it is possible to determine, in any of OB-fold or SH3 domain, to which amino acids correspond the amino acids as disclosed below for Sac7d.

### Description of variants based on the consensus sequence of the Sac7d family (SEQ ID NO: 16)

In sequences SEQ ID NO: 17-SEQ ID NO: 37, the amino acids designated as X or Xaa at positions 8-10, 22, 27, 30, 32, 42, 46 and 48 are as indicated in Table 2. The other amino acids designated as X (or Xaa) are indicated in Table 1 or Table 10 (where "-" designates no amino acid).

**Table 1. description of amino acids in SEQ ID NO: 17-SEQ ID NO: 37**

| **Position** | **Amino acid** |
|---|---|
| 2 | X is V, A or T |
| 3 | X is T or K |
| 4 | X is - or K |
| 6 | X is R or K |
| 15 | X is E or Q |
| 18 | X is T or I |
| 31 | X is V or I |
| 37 to 39 | XXX is EGG or DN- |
| 57 | X is M or L |
| 58 | X is Q, D or E |
| 59 | X is M or K |
| 61 to 67 | -------K, EKS--GKK, EK---QKK, ARAEREKK, ARAERE-K, ARAERE--, ARA-EKKK, E-----KK, EKS--GKK |

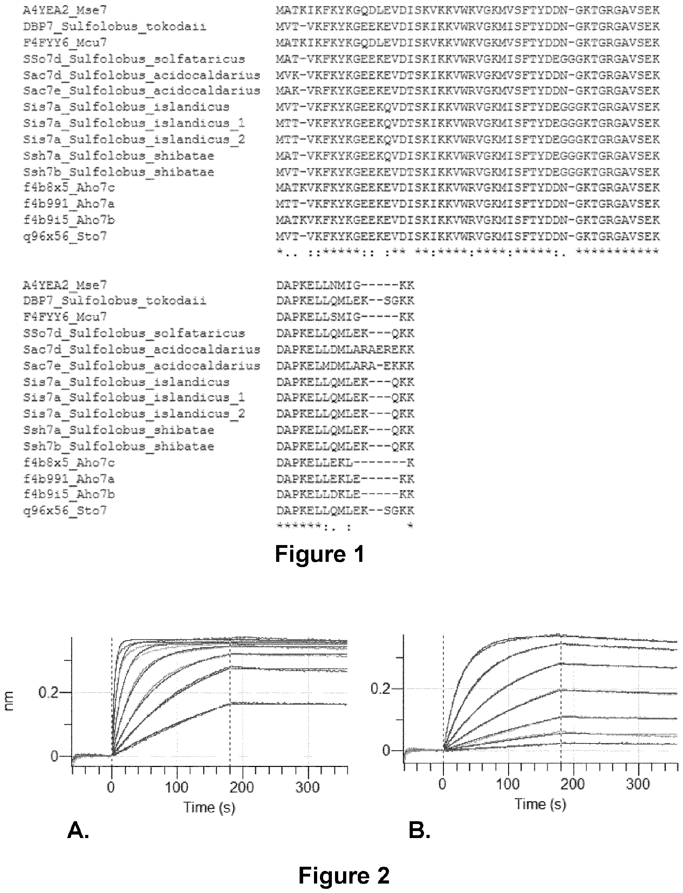

The amino acids disclosed in Table 1 correspond to the variability observed for proteins of the Sac7d family, as also shown in Figure 1.

The amino acids disclosed in Table 2 correspond to the amino acids that are involved in albumin binding, in the variants herein disclosed. It is to be noted that such variants further differ from the wild-type proteins for the following amino acids:
Position 23: presence of W; position 25: presence of L; position 34: presence of K; position 44: presence of Y.

These amino acids have been shown by the inventors as important for the binding to albumin (change of the amino acid drastically decreases the ability to bind to albumin or the affinity to the albumin). In contrast, it is possible to modify the other amino acids from the optimized binders (SEQ ID NO: 20, SEQ ID NO: 23, SEQ ID NO: 27, SEQ ID NO: 30, SEQ ID NO: 34 or SEQ ID NO: 37), according to Table 2. In particular it has been noted that replacement of the amino acids at positions 22, 27, 30, 32, 42 or 48 of these binders by another amino acids essentially didn't alter binding or its characteristics. With regards to amino acids at positions 8 and 10 in these binders, they can be changed respectively by hydrophobic/aromatic and polar/charged amino acids as listed in Table 2.

It has also been shown that the D17 (aspartic acid) of the consensus sequence (and which is not involved in binding) can be replaced by a E (glutamic acid). This is represented in the sequences mentioned above (SEQ ID NO: 20, SEQ ID NO: 23 bearing an X in position 17; SEQ ID NO: 27 and SEQ ID NO: 30 bearing a D17; SEQ ID NO: 34 and SEQ ID NO: 37 bearing a E17).

**Table 2. description of amino acids indicated as Xaa in SEQ ID NO: 17-SEQ ID NO: 37. It is to be noted that this table is to be used for the sequences mentioned above, for the Xaa that they bear. For some sequences, an amino acid is specified at some of the positions of Table 2 and the line of the table is thus not applicable.**

| Position | Amino-acid | Preferred 1 (B11) | Preferred 2 (F06) |
|---|---|---|---|
| 8 | Xaa is W,F,Y,V,I,L | W | V |
| 9 | Xaa is P or F | P | F |
| 10 | Xaa is R,K,S,T,Q,N | R | S |
| 22 | Xaa is any amino acid | A | K |
| 27 | Xaa is any amino acid | A | W |
| 30 | Xaa is any amino acid | T | A |
| 32 | Xaa is any amino acid | M | G |
| 42 | Xaa is any amino acid | K | I |
| 46 | Xaa is V or F | V | F |
| 48 | Xaa is any amino acid | L | A |

As indicated above, the methionine at position 1 of SEQ ID NO: 17-37 can be omitted. So can amino acids 61-68 of SEQ ID NO: 17-37.

In one embodiment, the polypeptide comprises SEQ ID NO: 17 or amino acids 2-60 of one of the sequence from Table 3.

**Table 3. List of variants sequences based on the consensus sequence of the proteins of the Sac7d family. The nature of X is provided in Tables 1 and 2. X at position 17 can be E or D.**

| SEQ ID NO: | Sequence |
|---|---|
| 17 | MXXXVXFXXXGEEKXVXXSKIXWVLRXGKXXXFKYDXXXGKXG YGXVXEKDAPKELXXXLXXXXXXXX |
| 18 | MXXXVXFXFXGEEKXVXXSKIXWVLRXGKXXXFKYDXXXGKXG YGFVSEKDAPKELXXXLXXXXXXXX |
| 19 | MXXXVXFVFSGEEKXVXXSKIXWVLRXGKXXXFKYDXXXGKXG YGFVXEKDAPKELXXXLXXXXXXXX |
| 20 | MXXXVXFVFSGEEKXVXXSKIKWVLRWGKAXGFKYDXXXGKIG YGFVAEKDAPKELXXXLXXXXXXXX |
| 21 | MXXXVXFXPXGEEKXVXXSKIXWVLRXGKXXXFKYDXXXGKXG YGVVSEKDAPKELXXXLXXXXXXXX |
| 22 | MXXXVXFWPRGEEKXVXXSKIXWVLRXGKXXXFKYDXXXGKX GYGVVXEKDAPKELXXXLXXXXXXXX |
| 23 | MXXXVXFWPRGEEKXVXXSKIAWVLRADKTXMFKYDXXXGKK GYGVVLEKDAPKELXXXLXXXXXXXX |
| 24 | MXXXVXFXXXGEEKXVDXSKIXWVLRXGKXXXFKYDXXXGKXG YGXVXEKDAPKELXXXLXXXXXXXX |
| 25 | MXXXVXFXFXGEEKXVDXSKIXWVLRXGKXXXFKYDXXXGKXG YGFVSEKDAPKELXXXLXXXXXXXX |
| 26 | MXXXVXFVFSGEEKXVDXSKIXWVLRXGKXXXFKYDXXXGKXG YGFVXEKDAPKELXXXLXXXXXXXX |
| 27 | MXXXVXFVFSGEEKXVDXSKIKWVLRWGKAXGFKYDXXXGKIG YGFVAEKDAPKELXXXLXXXXXXXX |
| 28 | MXXXVXFXPXGEEKXVDXSKIXWVLRXGKXXXFKYDXXXGKXG YGVVSEKDAPKELXXXLXXXXXXXX |
| 29 | MXXXVXFWPRGEEKXVDXSKIXWVLRXGKXXXFKYDXXXGKX GYGVVXEKDAPKELXXXLXXXXXXXX |
| 30 | MXXXVXFWPRGEEKXVDXSKIAWVLRADKTXMFKYDXXXGKK GYGVVLEKDAPKELXXXLXXXXXXXX |
| 31 | MXXXVXFXXXGEEKXVEXSKIXWVLRXGKXXXFKYDXXXGKXG YGXVXEKDAPKELXXXLXXXXXXXX |
| 32 | MXXXVXFXFXGEEKXVEXSKIXWVLRXGKXXXFKYDXXXGKXG YGFVSEKDAPKELXXXLXXXXXXXX |
| 33 | MXXXVXFVFSGEEKXVEXSKIXWVLRXGKXXXFKYDXXXGKXG YGFVXEKDAPKELXXXLXXXXXXXX |
| 34 | MXXXVXFVFSGEEKXVEXSKIKWVLRWGKAXGFKYDXXXGKIG YGFVAEKDAPKELXXXLXXXXXXXX |
| 35 | MXXXVXFXPXGEEKXVEXSKIXWVLRXGKXXXFKYDXXXGKXG YGVVSEKDAPKELXXXLXXXXXXXX |
| 36 | MXXXVXFWPRGEEKXVEXSKIXWVLRXGKXXXFKYDXXXGKX GYGVVXEKDAPKELXXXLXXXXXXXX |
| 37 | MXXXVXFWPRGEEKXVEXSKIAWVLRADKTXMFKYDXXXGKK GYGVVLEKDAPKELXXXLXXXXXXXX |

### Description of variant based on Sac7d and Aho7c

Sac7d and Aho7c are proteins that have large similarity.

SEQ ID NO: 87 corresponds to the consensus sequence of after alignment of amino acids 2-66 of Sac7d (SEQ ID NO: 1) and 3-60 of Aho7c (SEQ ID NO: 14). The starting amino acids have been omitted as they are not essential in the structure of the proteins.

XKVKFKYKGEEKEVDXSKIKKVWRVGKMXSFTYDDNGKTGRGAVSEKDAPK
ELLXXXXXXXXXX (SEQ ID NO: 87)

In this consensus sequences, X (or Xaa) are as in Table 4.

**Table 4. Description of the amino acids represented as Xaa in bEQ ID NO: 87 and SEQ ID NO: 38-44.**

| Position | Amino-acid | Preferred 1 Sac7d | Preferred 2 Aho7c |
|---|---|---|---|
| 1 | V or T | V | T |
| 16 | T or I | T | I |
| 29 | V or I | V | I |
| 55-64 | DMLARAEREK or EKLK---- | DMLARAEREK | EKLK |

### Such variants are represented by

**Table 5: sequences of the variants based on the consensus sequence of Sac7d-Aho7c. Amino acids represented by X are further described in Tables 4, 6 and 7**

| SEQ ID NO: | Sequence |
|---|---|
| 38 | XKVKFXXXGEEKEVXXSKIXWVLRXGKXXXFKYDDXGKXGYGX VXEKDAPKELLXXXXXXXXXX |
| 39 | XKVKFXFXGEEKEVXXSKIXWVLRXGKXXXFKYDDXGKXGYGF VXEKDAPKELLXXXXXXXXXX |
| 40 | XKVKFVFSGEEKEVXXSKIXWVLRXGKXXXFKYDDXGKXGYGF VXEKDAPKELLXXXXXXXXX |
| 41 | XKVKFVFSGEEKEVXXSKIKWVLRWGKAXGFKYDDXGKIGYGF VAEKDAPKELLXXXXXXXXXX |
| 42 | XKVKFXPXGEEKEVXXSKIXWVLRXGKXXXFKYDDXGKXGYGV VXEKDAPKELLXXXXXXXXXX |
| 43 | XKVKFWPRGEEKEVXXSKIXWVLRXGKXXXFKYDDXGKXGYG VVXEKDAPKELLXXXXXXXXXX |
| 44 | XKVKFWPRGEEKVVXXSKIAWVLRADKTXMFKYDDXGKKGYG VVLEKDAPKELLXXXXXXXXXX |

**Table 6. description of amino acids indicated as Xaa in SEQ ID NO: 38-SEQ ID NO: 86. It is to be noted that this table is to be used for the sequences mentioned above, for the Xaa that they bear. For some sequences, an amino acid is specified at some of the positions of Table 6 and the line of the table is thus not applicable.**

| Position | Amino-acid | Preferred 1 (B11) | Preferred 2 (F06) |
|---|---|---|---|
| 6 | Xaa is W,F,Y,V,I or L | W | V |
| 7 | Xaa is P or F | P | F |
| 8 | Xaa is R,K,S,T,Q or N | R | S |
| 20 | Xaa is any amino acid | A | K |
| 25 | Xaa is any amino acid | A | W |
| 28 | Xaa is any amino acid | T | A |
| 30 | Xaa is any amino acid | M | G |
| 39 | Xaa is any amino acid | K | I |
| 43 | Xaa is V or F | V | F |
| 45 | Xaa is any amino acid | L | A |

In these sequences, some amino acids that are not involved in binding can also be modified on the variants (see in particular SEQ ID NO: 38-SEQ ID NO: 86). They are represented in Table 7.

**Table 7. Amino acids represented as Xaa in SEQ ID NO: 36-44, SEQ ID NO: 45-51 and SEQ ID NO: 66-72. Xaa at position 56 is only present in SEQ ID 45-51.**

| Position | Amino-acid |
|---|---|
| 15 | Xaa is D or E |
| 36 | Xaa is N or Q |
| 56 | Xaa is M or L |

### Description of variants based on Sac7d (SEQ ID NO: 1)

Variants of Sac7d, binding to human and mouse serum albumin are depicted by SEQ ID NO: 45 to SEQ ID NO: 65.

In these sequences, the starting methionine (M) of the Sac7d protein has been omitted. It has indeed been shown by the Applicant that the activity of the proteins is not modified when this amino acid is deleted. Likewise, it is possible to omit the last 7 amino acids from the variants and retain activity.

Consequently, proteins depicted by amino acids 1-57 of any of SEQ ID NO: 45 to SEQ ID NO: 65 are also variants according to the invention. One can also cite proteins depicted by amino acids 1-58, 1-59, 1-60, 1-61, 1-62, 1-63 of any of SEQ ID NO: 45 to SEQ ID NO: 65, which are also variants according to the invention.

Consequently, the polypeptide comprise any of SEQ ID NO: 45 to SEQ ID NO: 65, or any truncated protein based on these sequences, and as disclosed above.

Such variants are represented by

**Table 8: sequences of the variants based on Sac7d. Amino acids represented by X are further described in Tables 6 and 7.**

| SEQ ID NO: | Sequence |
|---|---|
| 45 | VKVKFXXXGEEKEVXTSKIXWVLRXGKXVXFKYDDXGKXGYGX VXEKDAPKELLDXLARAEREK |
| 46 | VKVKFXFXGEEKEVXTSKIXWVLRXGKXVXFKYDDXGKXGYGF VXEKDAPKELLDXLARAEREK |
| 47 | VKVKFVFSGEEKEVXTSKIXWVLRXGKXVXFKYDDXGKXGYGF VXEKDAPKELLDXLARAEREK |
| 48 | VKVKFVFSGEEKEVXTSKIKWVLRWGKAVGFKYDDXGKIGYGF VAEKDAPKELLDXLARAEREK |
| 49 | VKVKFXPXGEEKEVXTSKIXWVLRXGKXVXFKYDDXGKXGYGV VXEKDAPKELLDXLARAEREK |
| 50 | VKVKFWPRGEEKEVXTSKIXWVLRXGKXVXFKYDDXGKXGYG VVXEKDAPKELLDXLARAEREK |
| 51 | VKVKFWPRGEEKVVXTSKIAWVLRADKTVMFKYDDXGKKGYG VVLEKDAPKELLDXLARAEREK |
| 52 | VKVKFXXXGEEKEVDTSKIXWVLRXGKXVXFKYDDNGKXGYGX VXEKDAPKELLDMLARAEREK |
| 53 | VKVKFXFXGEEKEVDTSKIXWVLRXGKXVXFKYDDNGKXGYGF VXEKDAPKELLDMLARAEREK |
| 54 | VKVKFVFSGEEKEVDTSKIXWVLRXGKXVXFKYDDNGKXGYGF VXEKDAPKELLDMLARAEREK |
| 55 | VKVKFVFSGEEKEVDTSKIKWVLRWGKAVGFKYDDNGKIGYGF VAEKDAPKELLDMLARAEREK |
| 56 | VKVKFXPXGEEKEVDTSKIXWVLRXGKXVXFKYDDNGKXGYGV VXEKDAPKELLDMLARAEREK |
| 57 | VKVKFWPRGEEKEVDTSKIXWVLRXGKXVXFKYDDNGKXGYG VVXEKDAPKELLDMLARAEREK |
| 58 | VKVKFWPRGEEKVVDTSKIAWVLRADKTVMFKYDDNGKKGYG VVLEKDAPKELLDMLARAEREK |
| 59 | VKVKFXXXGEEKEVETSKIXWVLRXGKXVXFKYDDQGKXGYGX |
| | VXEKDAPKELLDLLARAEREK |
| 60 | VKVKFXFXGEEKEVETSKIXWVLRXGKXVXFKYDDQGKXGYGF VXEKDAPKELLDLLARAEREK |
| 61 | VKVKFVFSGEEKEVETSKIXWVLRXGKXVXFKYDDQGKXGYGF VXEKDAPKELLDLLARAEREK |
| 62 | VKVKFVFSGEEKEVETSKIKWVLRWGKAVGFKYDDQGKIGYGF VAEKDAPKELLDLLARAEREK |
| 63 | VKVKFXPXGEEKEVETSKIXWVLRXGKXVXFKYDDQGKXGYGV VXEKDAPKELLDLLARAEREK |
| 64 | VKVKFWPRGEEKEVETSKIXWVLRXGKXVXFKYDDQGKXGYG VVXEKDAPKELLDLLARAEREK |
| 65 | VKVKFWPRGEEKVVETSKIAWVLRADKTVMFKYDDQGKKGYG VVLEKDAPKELLDLLARAEREK |

As indicated above, it is possible to modify D16 of Sac7d (which is located at position 15 in SEQ ID NO: 45-65, as the M1 present in Sac7d has been omitted), N37 of Sac7d (herein located at position 36) or M57 of Sac7d (herein located at position 56) as disclosed in Table 7.

As for the consensus sequence of Sac7d / Aho7c, any combination of substitution is foreseen (numbering is with respect to SEQ ID NO: 45-65), even though the sequence listing doesn't all depict them:

| | | | |
|---|---|---|---|
| D15 | N36 | M56 | SEQ ID NO: 52-58 |
| D15 | N36 | L56 | |
| D15 | Q36 | M56 | |
| D15 | Q36 | L56 | |
| E15 | N36 | M56 | |
| E15 | N36 | L56 | |
| E15 | Q36 | M56 | |
| E15 | Q36 | L56 | SEQ ID NO: 59-65 |

As indicated above, the sequences all contain the mutated amino acids W21, L23, K32, Y41 (corresponding respectively to position 22, 24, 33 and 42 of SEQ ID NO: 1, which contain the N-terminus methionine), which differ from the amino acids that are naturally present in Sac7d.

The Applicant has indeed found that these amino acids are present in various variants binding to serum albumin, and that modification of such leads to decrease of binding (loss of affinity or loss of binding). The other amino acids can be variable, under conditions mentioned in Table 6.

Very interesting variants are depicted by SEQ ID NO: 47, SEQ ID NO: 48, SEQ ID NO: 50, SEQ ID NO: 51, SEQ ID NO: 54, SEQ ID NO: 55, SEQ ID NO: 57, SEQ ID NO: 58, SEQ ID NO: 61, SEQ ID NO: 62, SEQ ID NO: 64 and SEQ ID NO: 65.

In particular, very interesting variants are depicted by SEQ ID NO: 48, SEQ ID NO: 51, SEQ ID NO: 55, SEQ ID NO: 58, SEQ ID NO: 62, and SEQ ID NO: 65,

SEQ ID NO: 62 (named F06) is of particular interest, as well as SEQ ID NO: 55. It is to be noted that the L56 of SEQ ID NO: 62 can be replaced by M56.

SEQ ID NO: 58 (named B11) is also of particular interest, as well as SEQ ID NO: 65.

### Description of proteins based on Aho7c (SEQ ID NO: 14)

Variants of Aho7c, binding to human and mouse serum albumin are depicted by SEQ ID NO: 66 to SEQ ID NO: 86. As indicated above, such protein is very similar to Sac7d. It has also been shown and reminded herein that mutations of Sac7d can be carried from Sac7d to another protein (WO 2012/150314).

In these sequences, the starting methionine and alanine (MA) of the Aho7c protein (SEQ ID NO: 14) have been omitted. It has indeed been shown by the Applicant that the activity of the proteins is not modified when these amino acids are deleted. Likewise, it is possible to omit the last 4 amino acids from the variants and retain activity.

Consequently, proteins depicted by amino acids 1-55 of any of SEQ ID NO: 66 to SEQ ID NO: 86 are also variants according to the invention. One can also cite proteins depicted by amino acids 1-56, 1-57, 1-58 of any of SEQ ID NO: 66 to SEQ ID NO: 86, which are also variants according to the invention.

Consequently, the polypeptide comprise any of SEQ ID NO: 66 to SEQ ID NO: 86, or any truncated protein based on these sequences, and as disclosed above.

Such variants are represented by

**Table 9: sequences of the variants based on Ao7c. Amino acids represented by X are further described in Tables 6 and 7.**

| SEQ ID NO: | Sequence |
|---|---|
| 66 | TKVKFXXXGEEKEVXISKIXWVLRXGKXIXFKYDDXGKXGYGXV XEKDAPKELLEKLK |
| 67 | TKVKFXFXGEEKEVXISKIXWVLRXGKXIXFKYDDXGKXGYGFV XEKDAPKELLEKLK |
| 68 | TKVKFVFSGEEKEVXISKIXWVLRXGKXIXFKYDDXGKXGYGFV XEKDAPKELLEKLK |
| 69 | TKVKFVFSGEEKEVXISKIKWVLRWGKAIGFKYDDXGKIGYGFV AEKDAPKELLEKLK |
| 70 | TKVKFXPXGEEKEVXISKIXWVLRXGKXIXFKYDDXGKXGYGVV XEKDAPKELLEKLK |
| 71 | TKVKFWPRGEEKEVXISKIXWVLRXGKXIXFKYDDXGKXGYGVV XEKDAPKELLEKLK |
| 72 | TKVKFWPRGEEKVVXISKIAWVLRADKTIMFKYDDXGKKGYGVV LEKDAPKELLEKLK |
| 73 | TKVKFXXXGEEKEVDISKIXWVLRXGKXIXFKYDDNGKXGYGXV XEKDAPKELLEKLK |
| 74 | TKVKFXFXGEEKEVDISKIXWVLRXGKXIXFKYDDNGKXGYGFV XEKDAPKELLEKLK |
| 75 | TKVKFVFSGEEKEVDISKIXWVLRXGKXIXFKYDDNGKXGYGFV XEKDAPKELLEKLK |
| 76 | TKVKFVFSGEEKEVDISKIKWVLRWGKAIGFKYDDNGKIGYGFV AEKDAPKELLEKLK |
| 77 | TKVKFXPXGEEKEVDISKIXWVLRXGKXIXFKYDDNGKXGYGVV XEKDAPKELLEKLK |
| 78 | TKVKFWPRGEEKEVDISKIXWVLRXGKXIXFKYDDNGKXGYGV VXEKDAPKELLEKLK |
| 79 | TKVKFWPRGEEKVVDISKIAWVLRADKTIMFKYDDNGKKGYGV VLEKDAPKELLEKLK |
| 80 | TKVKFXXXGEEKEVEISKIXWVLRXGKXIXFKYDDQGKXGYGXV XEKDAPKELLEKLK |
| 81 | TKVKFXFXGEEKEVEISKIXWVLRXGKXIXFKYDDQGKXGYGFV XEKDAPKELLEKLK |
| 82 | TKVKFVFSGEEKEVEISKIXWVLRXGKXIXFKYDDQGKXGYGFV |
| | XEKDAPKELLEKLK |
| 83 | TKVKFVFSGEEKEVEISKIKWVLRWGKAIGFKYDDQGKIGYGFV AEKDAPKELLEKLK |
| 84 | TKVKFXPXGEEKEVETSKIXWVLRXGKXVXFKYDDQGKXGYGV VXEKDAPKELLEKLK |
| 85 | TKVKFWPRGEEKEVETSKIXWVLRXGKXVXFKYDDQGKXGYG VVXEKDAPKELLEKLK |
| 86 | TKVKFWPRGEEKVVETSKIAWVLRADKTVMFKYDDQGKKGYG VVLEKDAPKELLEKLK |

As indicated above, it is possible to modify D17 of Aho7c (which is located at position 15 in SEQ ID NO: 66-86, as the M1A2 present in Aho7c have been omitted), or N38 of Aho7c (herein located at position 36) as disclosed in Table 7.

As for the consensus sequence of Sac7d / Aho7c, any combination of substitution is foreseen (numbering is with respect to SEQ ID NO: 66-86), even though the sequence listing doesn't all depict them:

| | | |
|---|---|---|
| D15 | N36 | SEQ ID NO: 73-79 |
| D15 | Q36 | |
| E15 | N36 | |
| E15 | Q36 | SEQ ID NO: 80-86 |

As indicated above, the sequences all contain the mutated amino acids W21, L23, K32, Y41 (corresponding respectively to position 23, 25, 34 and 43 of SEQ ID NO: 14, which contain the methionine and alanine in N-terminus), which differ from the amino acids that are naturally present in Aho7c.

The Applicant has indeed found that these amino acids are present in various variants binding to serum albumin, and that modification of such leads to decrease of binding (loss of affinity or loss of binding). The other amino acids can be variable, under conditions mentioned in Table 6.

Very interesting variants are depicted by SEQ ID NO: 68, SEQ ID NO: 69, SEQ ID NO: 71, SEQ ID NO: 72, SEQ ID NO: 75, SEQ ID NO: 76, SEQ ID NO: 78, SEQ ID NO: 79, SEQ ID NO: 82, SEQ ID NO: 83, SEQ ID NO: 85 and SEQ ID NO: 86.

In particular, very interesting variants are depicted by SEQ ID NO: 69, SEQ ID NO: 72, SEQ ID NO: 76, SEQ ID NO: 79, SEQ ID NO: 83, and SEQ ID NO: 86,

SEQ ID NO: 83 (based on F06) is of particular interest.

SEQ ID NO: 86 (based on B11) is also of particular interest.

In a specific embodiment, the polypeptide consists in the variant of a protein of the Sac7d family binding to human serum albumin (HSA).

In a specific embodiment, the variant of a protein of the Sac7d family binding to HSA is linked or fused to another protein or polypeptide (herein also indicated as associated).

The invention also pertains to a genetic construct comprising a DNA sequence coding for the polypeptide described herein, to a vector comprising such genetic construct, and to a host cell comprising the genetic construct in its genome (the nucleic acid or the vector).

The invention also pertains to a method for producing the polypeptide herein disclosed, comprising the steps consisting of
a. Culturing a cell culture wherein the cells have been transformed by a genetic construct as disclosed,
   and
b. Recovering the polypeptide.

The invention also relates to a polypeptide herein disclosed as a medicament.

The invention also relates to a a fusion protein comprising a variant herein disclosed fused to a peptide or protein with therapeutic activity as a medicament.

The invention also relates to a method for treating a subject in need thereof comprising administering a therapeutic amount of a polypeptide herein disclosed to the subject, in particular when the subject has cancer, a metabolic disease (such as diabetes), a neurologic disease, an inflammatory or autoimmune disease (such as rheumatoid arthritis, psoriasis, lupus or multiple sclerosis), or enzyme deficiency in particular in the brain. The polypeptide that is administered to the subject comprises the variant of the protein of the Sac7d family, binding to human serum albumin, fused to a protein or peptide that has therapeutic activity against the disease that is to be treated. For instance, a protein that neutralizes Interleukin-17 for treating psoriasis, a protein that neutralizes Tumor Necrosis Factor alpha for the treatment of Rheumatoid Arthritis, a protein that prevents the interaction of Programmed Death Ligand 1 with its receptors on the surface of cancer cells, a protein that neutralizes stress resistance factors such as Heat Shock Proteins (in particular HSP 110).

A therapeutic amount (or effective amount) is an amount sufficient to effect beneficial or desired results, such as clinical results, and thus depends upon the context in which it is being applied. In the context of administering a polypeptide comprising the variant binding to HSA and a peptide or polypeptide having a therapeutic activity, to a patient with a disease herein disclosed, an effective amount of an agent is, for example, an amount sufficient to achieve cure of the patient, improvement of the patient's condition, acceleration of the patient's cure, as compared to the response obtained without administration of the agent. It can also be an amount that stops or slows the progression of the disease. It can be an amount that allows regression of the disease, or of biological markers associated with the specific disease.

An agent with therapeutic activity (or a therapeutic agent) is an agent which, upon administration at an efficient amount to a subject having a disease improves the condition of the subject, or decreases a biomarker linked to the disease.

It is reminded that the sequence of Sac7d is:

MVKVKFKYKGEEKEVDTSKIKKVWRVGKMVSFTYDDNGKTGRGAVSEKDAPKEL
LDMLARAEREKK (SEQ ID NO: 1).

The variants binding to HSA herein disclosed contain mutations at positions corresponding to the positions 7, 8, 9, 21, 22, 24, 26, 29, 31, 33, 40, 42, 44 and 46 of the Sac7 sequence (positions 6, 7, 8, 20, 21, 23, 25, 28, 30, 32, 39, 41, 43 and 45 of SEQ ID NO: 38-86). The lysine at position 21 in SEQ ID NO 1 may be maintained in some variants. The lysine at position 8 in SEQ ID NO 1may also be maintained in some variants, the native amino acids at positions 26, 29 31, 40 or 46 in SEQ ID NO 1 or the phenylalanine at position 43 in SEQ ID NO 1.

However, the variant contains at least 4 mutated amino acids as compared to the native sequence which are the W22, L24, K33 and Y42 in SEQ ID NO 1 (W21, L23, K32 and Y41 in SEQ ID NO 38-86), preferably at least 6, more preferably at least 9, or at least or exactly 12 mutated amino acids.

It is preferred when the variant of a protein of the Sac7d family binding to HSA is linked or fused to another protein or polypeptide.

It may be bound our fused to another variant of a protein of the Sac7d family, to a peptide, or to an antibody (as described in WO2019096797).

In particular, the other variant of the protein of the Sac7d family may bind to IL-17 (see WO2019096797), or to TNF-alpha (see WO2020074402).

In another embodiment, the polypeptide is conjugated to an organic molecule.

It also allows obtaining a genetic construct comprising a DNA sequence coding for a polypeptide defined above, a vector comprising such genetic construct and a host cell comprising such genetic construct in its genome. Such vector also includes the elements allowing transcription and translation of the polypeptide (such as promoters, terminators, enhancers...).

It is possible to perform a method for producing the polypeptide, comprising culturing a cell culture wherein the cells have been transformed by a genetic construct, and recovering the polypeptide.

One can also embody a polypeptide as disclosed as a medicament, in particular a polypeptide comprising the variant binding to HAS fused with another peptide or polypeptide having therapeutic activity..

It is to be noted that the sequences indicated above may comprise a methionine at its N-terminal, but that it is also possible to perform the invention when such methionine listed at the N-terminal end of each sequence is not included. Similarly, the amino acids located at the end-terminus of the proteins may be omitted. In particular the amino acids located after residue L58 of Sac7d (residues located after L60 of SEQ ID NO: 16) may be omitted.

It is to be noted that the specification discloses variants of Sac7d, as an illustration, but the teachings are applicable to the other proteins of the Sac7d family. The teachings are also applicable to other OB-fold domains as disclosed in WO2007139397. In particular, mention is made of WO2012150314 which demonstrates that it is possible to carry mutations from one sequence of the Sac7d family to another one, using the alignment shown in Figure 1. The invention is also applicable to SH3 domains, a small protein domain of about 60 amino acid residues, initially, described as a conserved sequence in the viral adaptor protein v-Crk and described under PF00018 in the PFAM database. The SH3 domain has a characteristic beta-barrel fold that consists of five or six β-strands arranged as two tightly packed anti-parallel β sheets. The linker regions may contain short helices. It is to be noted that OB-fold and SH3 domains share homology and that, in view of the knowledge of the sequence and structure of these domains, it is possible to determine, in any of OB-fold or SH3 domain, to which amino acids correspond the amino acids as disclosed below for Sac7d.

### The Sac7d protein family

The Sac7d family is defined as relating to the Sac7d protein and corresponds to a family of 7 kDa DNA-binding proteins isolated from extremophilic bacteria. It is herein disclosed as a representative species of OB-fold domains, that is preferably used in the context of the invention. Since SH3 domains share homology with OB-fold domains, the teachings pertaining to Sac7d are also applicable to SH3 scaffolds.

These proteins and this family are in particular described in WO 2008/068637. Thus, within the context of the present invention, a protein belongs to the Sac7d family when it has one of the sequences SEQ ID NO: 1 to SEQ ID NO: 15, or when it has a sequence corresponding to the sequence SEQ ID NO: 16 which is a consensus sequence (obtained from SEQ ID NO: 1 to SEQ ID NO: 9 and SEQ ID NO: 12 to SEQ ID NO: 15. In this consensus sequence, a dash - indicates no amino acid, the proteins not having all the same size). This Sac7d family comprises in particular the Sac7d or Sac7e proteins derived from *Sulfolobus acidocaldarius,* the Sso7d protein derived from *Sulfolobus solfataricus,* the DBP 7 also called Sto7 protein derived from *Sulfolobus tokodaii,* the Ssh7b protein derived from *Sulfolobus shibatae,* the Ssh7a protein derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and the p7ss protein derived from *Sulfolobus solfataricus.* In view of the broad similarity of the sequences of the proteins of the Sac7d family, it is directly possible and easy to identify the amino acids of another protein than Sac7d that correspond to given amino acids of Sac7d. In particular, one can also use the teachings of WO 2008/068637 that shows (in the figures) and explains (in the specification) that such proteins are superimposable.

WO 2012/150314 shows that the mutations from one protein of the Sac7d family can be carried to another protein of the same family. This portability amounts to creating a mutant of another protein of the Sac7d family, starting from a mutant of one protein of said family. The first mutant may have been obtained in particular by carrying out the process of WO 2008/068637. Consequently, it is possible, using in particular the teachings of WO 2012/150314 and of figure 1, to obtain a mutant of any protein of the Sac7d family, starting from a mutant of any other protein of such family. As an illustration, for a mutant of Sac7d, one can introduce the mutated amino acids of the Sac7d mutant within the scaffold of another protein, using the sequence alignment of figure 1. As an illustration, variants of Aho7c, obtained from the Sac7d variants herein disclosed, are described as SEQ ID NO: 66 to SEQ ID NO: 86.

The number of mutated residues introduced within a wild-type protein sequence to obtain a variant is preferably between 4 and 25, or more specifically between 4 and 22. It is thus possible to obtain variants preferably having at least 4, more preferably at least 5, more preferably at least 6, more preferably at least 7 or 8, even more preferably at least 10, but generally less than 25, more preferably less than 22, even more preferably less than 20 or less than 15 or 14 substituted amino acids compared with the wild-type OB-fold protein (or domain). It is to be noted that all and any ranges are considered herein, (such as 5-20 or 7-25 and so forth). Particularly preferred ranges are 4-17 and 6-17, 4-11, 4-12, 5-12, 6-12, 4-14 and 6-14.

It is preferred when 7, 8, 9, 10, 11, 12, 13 or 14 amino acids are mutated in the binding site of the OB-fold domain, relative to the wild-type OB-fold domain. These mutations are introduced in the amino acids corresponding to V2, K3, K5, K7, Y8, K9, G10, E11, K13, E14, T17, K21, K22, W24, V26, G27, K28, M29, S31, T33, Y34, D36, N37, G38, K39, T40, R42, A44, S46, E47, K48, D49, A50 and P51 of Sac7d (SEQ ID NO: 1), preferably in the amino acids corresponding to K7, Y8, K9, K21, K22, W24, V26, M29, S31, T33, T40, R42, A44, and S46.

In particular, it is interesting when the number of mutated amino acids is between 7 and 14 (limits included). In particular, the variants can also comprise amino acid insertions as indicated above.

As indicated, proteins of the Sac7d family are Sac7d or Sac7e derived from *Sulfolobus acidocaldarius,* Sso7d derived from *Sulfolobus solfataricus,* DBP 7 also called Sto7 derived from *Sulfolobus tokodaii,* Ssh7b derived from *Sulfolobus shibatae,* Ssh7a derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and p7ss derived from *Sulfolobus solfataricus.* The various sequences of the Sac7d, Sso7d, Sac7e, Ssh7b, Ssh7a, DBP7, Sis7a (3 alleles), Mse7, Mcu7, Aho7a, Aho7b, Aho7c and Sto7d proteins are represented by SEQ ID NO: 1 to SEQ ID NO: 15 respectively.

A variant of a protein of this Sac7d family may be called a nanofitin. The invention is thus preferentially implemented on variants of the proteins represented by SEQ ID NO: 1 to SEQ ID NO: 15, or a protein having the sequence SEQ ID NO: 16, in particular on variants of Sac7d.

### Link of Sac7d protein with OB-fold proteins and SH3 domains

The OB-fold proteins are known in the art. They are in particular described in the documents cited above, and also in Arcus (Curr Opin Struct Biol. 2002 Dec; 12(6):794-801). OB-fold is in the form of a cylinder having five beta (β) sheets. Most OB-fold proteins use the same binding interface of their natural ligand, which may be an oligosaccharide, an oligonucleotide, a protein, a metal ion or a catalytic substrate. This binding interface comprises mainly the residues located in the beta sheets. Certain residues located in the loops may also be involved in the binding of an OB-fold protein with its natural ligand. Thus, applications WO 2007/139397 and WO 2008/068637 and the Arcus document (2002, op. cit.) describe the OB-fold-protein domains for binding with their natural ligand.

In particular, document WO 2008/068637 describes precisely how to identify the binding domain of an OB-fold protein. By superimposing several sequences and 3D structures of proteins having OB-fold domains, using the websites WU-Blast2 (http://www.ebi.ac.uk/blast2/index.html) (Lopez et al., 2003, Nucleic Acids Res 31, 3795-3798), T-COFFEE (http://www.ch.embnet.org/software/TCoffee.html) (Notredame et al., 2000, J Mol Biol 302, 205-217) and DALI lite (http://www.ebi.ac.uk/DaliLite/) (Holm and Park, 2000, Bioinformatics 16, 566-567), it is possible to identify the positions of the binding domains and in particular the amino acids which can be modified. Taking the sequence of Sac7d (SEQ ID NO: 1) as a reference, these are the residues V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51, preferably K7, Y8, K9, K21, K22, W24, V26, M29, S31, T33, T40, R42, A44, and S46. Using the alignment presented in Figure 1, it is possible to identify, in each protein, the amino acids corresponding to these amino acids of Sac7d.

WO 2008/068637 describes that it is possible to perform a superimposition of 3D structures of OB-fold proteins or domains (10 domains were used in this application, including Sac7d), using the DALI website (http://www.ebi.ac.uk/dali/interactive.html)(Holm and Sander, 1998, Nucleic Acids Res 26, 316-319). Thus, it is easy to identify, for any OB-fold protein (or any OB-fold domain), the amino acids involved in the binding site and corresponding to the Sac7d amino acids mentioned above. Consequently, providing the amino acids that can be mutated in one of these proteins makes it possible to identify the corresponding amino acids for any other OB-fold domain.

It is also to be noted that OB-fold domains resemble SH3 domains and that it is also possible to identify equivalents of amino acids of Sac7d in SH3 domains.

### Example of OB-fold or SH3 domain

Non-limitative examples of OB-fold proteins which can be used according to the invention are Sac7d, Sso7d, the N-terminal domain of SEB (Papageorgiou et al., 1998), the chain A of the Shiga-like toxin Ile (PDB 2bosa), the human Neutrophil Activatin Peptide-2 (NAP-2, PDB 1tvxA), the Molybdenum Binding Protein (modg) of Azotobacter vinelandii (PDB 1h9j), the N-terminal domain of SPE-C (Roussel et al., 1997), the B5 subunit of E. coli Shiga-like toxin (Kitov et al., 2000), Cdc13 (Mitton-Fry et al., 2002), the cold-shock DNA-binding domain of the human Y-box protein YB-1 (Kloks et al., 2002), the E. coli inorganic pyrophosphatase EPPase (Samygina et al., 2001), or any of the proteins listed in Table 3 of the article by (Arcus, 2002), such as 1krs (Lysyl-tRNA synthetase LysS, E.coli), 1c0aA (Asp- tRNA synthetase, E.coli), 1b8aA (Asp- tRNA synthetase, P. kodakaraensis), 1lylA (Lysyl-tRNA synthetase LysU, E.coli), 1quqA (Replication protein A, 32kDa subunit, Human), 1quqB (Replication protein A, 14kDa subunit, Human), 1jmcA (Replication protein A, 70kDa subunit (RPA70) fragment, Human), 1otc (Telomere-end-binding protein, O. nova), 3ullA (Mitochondrial ssDNA-binding protein, Human), 1prtF (Pertussis toxin S5 subunit, B. pertussis), 1bcpD (Pertussis toxin S5 subunit (ATP bound), B. pertussis), 3chbD (Cholera Toxin, V. cholerae), 1tiiD (Heat-labile toxin, E. coli), 2bosA (Verotoxin-1/Shiga toxin, B-pentamer, E. coli), 1br9 (TIMP-2, Human), 1an8 (Superantigen SPE-C, S. pyogenes), 3seb (Superantigen SPE, S. aureus), 1aw7A (Toxic shock syndrome toxin, S. aureus), 1jmc (Major cold-shock protein, E.coli), 1bkb (Initiation translation factor 5a, P. aerophylum), 1sro (S1 RNA-binding domain of PNPase, E.coli), 1d7qA (Initiation translation factor 1, elF1a, Human), 1ah9 (Initiation translation factor 1, IF1, E.coli), 1b9mA (Mo-dependent transcriptional regulator ModE, E.coli), 1ckmA (RNA guanylyltransferase, Chlorella virus, PBCV-1), 1a0i (ATP-dependent DNA ligase, Bacteriophage T7), 1snc (Staphylococcal nuclease, S. aureus), 1hjp (DNA helicase RuvA subunit, N-terminal domain, E.coli), 1pfsA (Gene V protein, Pseudomonas bacteriophage pf3), 1gvp (Gene V protein, Filamentous bacteriophage (f1, M13)), 1gpc (Gene 32 protein (gp32) core, Bacteriophage T4), 1wgjA (Inorganic pyrophosphatase, S. cerevisiae), and 2prd (Inorganic pyrophosphatase, T. thermophilus).

Non-exhaustive examples of proteins with SH3 domaines are Signal transducing adaptor proteins, CDC24, Cdc25, PI3 kinase, Phospholipase, Ras GTPase-activating protein, Vav proto-oncogene, GRB2, p54 S6 kinase 2 (S6K2), SH3D21, C10orf76 (potentially), STAC3, Some myosins, SHANK1,2,3, ARHGAP12, C8orf46, TANGO1, Integrase, Focal Adhesion Kinase (FAK, PTK2), Proline-rich tyrosine kinase (Pyk2, CADTK, PTK2beta), or TRIP10 (cip4).

### Production of the identified variants

The sequence of the identified variant can be cloned in any appropriate vector by any molecular genetic methods known in the art.

These recombinant DNA constructs comprising a nucleotide sequence, coding for a polypeptide comprising a variant as described above, are used in connection with a vector, such as a plasmid, phagemid, phage or viral vector.

These recombinant acid molecules can be produced by techniques described in Sambrook et al., 1989 (Sambrook J, Fritschi EF and Maniatis T (1989) Molecular cloning: a laboratorymanual, Cold Spring Harbor Laboratory Press, New York). Alternatively, the DNA sequences may be chemically synthesized using, for example, synthesizers.

Recombinant constructs of the invention comprise the expression vectors that are capable of expressing the RNA and thus lead to production of proteins from the above genetic sequences. The vector may thus further comprise regulatory sequences, including a suitable promoter operably linked to the open reading frame (ORF) of the genetic sequences herein disclosed. The vector may further comprise a selectable marker sequence such as an antibiotic resistance gene. Specific initiation and bacterial secretory signals also may be required for efficient translation of the coding sequences when bacteria as used as the expression host.

### Production of the molecules

Cells are transfected or transformed with vectors containing the sequences coding for the polypeptides comprising the variant as disclosed above.

The cells are the cultured in such conditions as to have the protein expressed and favorably secreted. The conditions of culture of the cells are the conditions generally used for recombinant antibody production and are known in the art. Such conditions that are known in the art can also be optimized by the person skilled in the art if needed. Kunert and Reinhart (Appl Microbiol Biotechnol. 2016; 100: 3451-3461) review such methods and provide ample references thereto.

One can use bacterial, phage (Shukra et al, Eur J Microbiol Immunol (Bp). 2014; 4(2): 91-98) or eukaryotic systems of production.

One shall prefer to use eukaryotic cells in order to obtain proper post-translational modifications such as glycosylation.

In particular, one can use CHO (Chinese Hamster Ovary) cells, PER.C6 cells (human cell line, Pau et al, Vaccine. 2001 21;19(17-19):2716-21), HEK 293b cells (Human embryonic kidney cells 293), NS0 cells (cell line derived from the non-secreting murine myeloma) or EB66 cells (a duck cell line Valneva, Lyons, France).

Also provided by the present disclosure are host cells containing at least one of the DNAs constructs coding for a polypeptide comprising the variant as disclosed herein. The host cell can be any cell for which expression vectors are available. As indicated above, it may be a higher eukaryotic host cell, such as a mammalian cell, a lower eukaryotic host cell, such as a yeast cell, or a prokaryotic cell, such as a bacterial cell.

Introduction of the recombinant construct into the host cell is performed by any method known in the art (such as calcium phosphate transfection, lipofection, DEAE, dextran mediated transfection, electroporation or phage infection). The vectors can be inserted within the genome of the host cell, or be maintained as an extragenomic vector (such as a Bacterial Artificial Chromosome or a Yeast Artificial Chromosome). When introduced within the cell genome, such introduction may be random or targeted using methods known in the art (homologous recombination or the like).

### Bacterial hosts and expression

Useful expression vectors for bacterial use are constructed by inserting the recombinant DNA sequence together with suitable translation initiation and termination signals in operable reading phase with a functional promoter. The vector will comprise one or more phenotypic selectable markers and an origin of replication to ensure maintenance of the vector and, if desirable, to provide amplification within the host.

Suitable prokaryotic hosts for transformation include *E. coli, Bacillus subtilis, Salmonella typhimurium* and various species within the genera *Pseudomonas, Streptomyces,* and *Staphylococcus.*

### Eukaryotic hosts and expression

Examples of the eukaryotic host cells include vertebrate cells, insect cells, and yeast cells. In particular, one can use the cells mentioned above.

The transformed or transfected cells are cultured according to methods known in the art and the polypeptide are recovered from intracellular or extracellular fractions (depending on whether it is secreted or not).

### Isolation of the molecules

The recombinant protein produced can be separated and purified by any of various known separation methods utilizing the physical or chemical property of the protein, from the intracellular or extracellular fraction.

In particular, one can use methods such as precipitation, tangential flow filtration, ultrafiltration, various types of liquid chromatography such as molecular sieve chromatography (gel filtration), adsorption chromatography, ion exchange chromatography, and affinity chromatography, dialysis, and a combination thereof.

In general, any method known and used to purify recombinant polypeptides is adapted for the purification of the molecules herein disclosed.

If a tag has been introduced within the recombinant sequence (such as a poly-Histidine tag), one can purify the molecules using this tag. However, it is preferred to use affinity to purify the molecules.

One can, in particular, use the fact that the molecule herein produced binds to a specific target and use any affinity method (affinity column, FACS, beads) to isolate such molecules.

One particular advantage of the molecules herein disclosed is that they don't need to be glycosylated to be active and can thus be produced in any type of cells, and not necessarily eukaryotic cells. They are particularly well produced in bacterial cells.

### Modification of the variants

The variants described above, and having the ability to bind Human Serum Albumin as well as mouse Serum Albumin, can be modified by any method known in the art.

### Preparing a polypeptide comprising the variant

It is possible to prepare a DNA sequence that contains two coding sequences, one for the variant herein disclosed and another for a protein or peptide of interest (in particular with therapeutic activity). The resulting expressed protein is thus a polypeptide that contains both proteins. The vector may be built in such a way that it contains a sequence coding for a linker located between the two proteins in the expressed polypeptide.

Consequently, the invention also encompasses a polypeptide comprising the variant of a protein of the OB-fold protein (preferably of the Sac7d family) binding to HSA, which is linked (preferably through amine bound as disclosed above) to another protein or polypeptide.

In a specific embodiment, the other protein or polypeptide comprises another variant of a protein of the Sac7d family.

Are of particular interest
- A polypeptide comprising a variant of an OB-fold protein of the Sac7d family binding to HSA, fused with a variant of an OB-fold protein of the Sac7d family binding to Interleukin-17 (in N- or C-terminus) and neutralizing its interaction with its receptors for the treatment of autoimmune diseases such as Psoriasis or Psoriatic arthritis.
- A polypeptide comprising a variant of an OB-fold protein of the Sac7d family binding to HSA, fused with a variant of an OB-fold protein of the Sac7d family binding to Programmed Death Ligand 1 (in N- or C-terminus) and neutralizing its interaction with its cognate receptor for the treatment of cancer such as solid tumors (breast, lung, kidney, pancreatic cancers amongst other).
- A polypeptide comprising a variant of an OB-fold protein of the Sac7d family binding to HSA, fused with a variant of an OB-fold protein of the Sac7d family binding to Heat Shock Protein 110 (in N- or C-terminus) and neutralizing its ability to induce cell resistance to stress and the polarization of macrophages thereby alleviating resistance to anticancer drugs, and/or eliciting an anticancer activity on its own.
- A polypeptide comprising a variant of an OB-fold protein of the Sac7d family binding to HSA, fused with a variant of an OB-fold protein of the Sac7d family binding to Tumor Necrosis Factor alpha (in N- or C-terminus). Such polypeptide acts as an inhibitor of inflammatory signals mediated by TNFalpha and helps reduce inflammation in diseases such as Rheumatoid arthritis, Crohn's disease and more generally Inflammatory Bowel Diseases.

In another embodiment, the other protein or polypeptide is an antibody. In this embodiment, the variant of the OB-fold domain is fused to at least one of the heavy or light chain of an immunoglobulin monomer, preferably at the N- or C-terminus of the light or heavy chain. In another embodiment, the variant may be fused to both heavy or light chains.

In order to obtain such compounds, one can use a genetic construct comprising a DNA sequence selected from the group consisting of
a. the sequence coding for the heavy chain of an antibody fused, at its 3'end with the sequence coding for the variant of an OB-fold protein (potentially with an sequence coding for a linker)
b. the sequence coding for the heavy chain of an antibody fused, at its 5' end with the sequence coding for the variant of an OB-fold protein (potentially with an sequence coding for a linker)
c. the sequence coding for the light chain of an antibody fused, at its 3' end with the sequence coding for the variant of an OB-fold protein (potentially with an sequence coding for a linker)
d. the sequence coding for the light chain of an antibody fused, at its 5' end with the sequence coding for the variant of an OB-fold protein (potentially with an sequence coding for a linker).

This fusion can be made at the N-terminus and/or the C-terminus of the antibody chain (heavy and/or light chain). It is to be noted that, especially when using a small OB-fold domain (about 70 amino acids) such as a protein from the Sac7d family, it is possible to obtain a molecule that will have the structure of the antibody (two light chains paired to two heavy chains, and such dimers paired together), with the antibody regions, and further binding regions consisting of the modified OB-fold domain.

In a specific embodiment, the antibody part of the protein herein disclosed is a IgG molecule.

In another embodiment, the antibody part of the protein herein disclosed is a IgA molecule.

In another embodiment, the antibody part of the protein herein disclosed is a IgM molecule.

In another embodiment, the antibody part of the protein herein disclosed is a IgD molecule.

In another embodiment, the antibody part of the protein herein disclosed is a IgE molecule.

The antibody may be a human antibody, a rodent antibody (such as a mouse antibody or a rat antibody), a cat antibody, a dog antibody, a chicken antibody, a goat antibody, a camelid antibody (such as a camel antibody, a llama antibody, an alpaca antibody or a nanobody), a shark antibody, or an antibody from any other species. It may be a chimeric or humanized antibody. As reminded in Wikipedia, humanized antibodies are antibodies from non-human species whose protein sequences have been modified to increase their similarity to antibody variants produced naturally in humans. A chimeric antibody contains sequences from different species.

It is preferred when the antibody that is part of the molecule herein disclosed is an antibody that comprises two identical heavy chains (of about 400 to 500 amino acids, generally around 450 amino acids) and two identical light chains. Consequently, the antibody comprises the same Fab variable regions. This antibody is therefore a monospecific antibody where both parts (combination of light and heavy chains) of the antibody bind to the same epitope of an antigen.

However, the antibody may present different heavy and/or light chains. In particular, in some embodiments, the antibody is a bi-specific antibody. The term "antibody" thus encompasses both "classical antibodies" as disclosed above having identical heavy and light chains, but also engineered antibodies that have more than one specificity.

In a specific embodiment, the antibody presents one heavy and light chain from one antibody and another heavy and light chain from another antibody.

The antibody may bind to a target selected in the group consisting of:
Cell surface receptors: Insulin receptor, Low density lipoprotein receptor-related protein 1, Transferrin receptor, Epidermal growth factor receptor, Epidermal growth factor receptor variant III, Vascular endothelial growth factor receptor 1, Vascular endothelial growth factor receptor 2, Her2, Her3, Her4, PMSA, IGF-1R, GITR, RAGE, CD28.

Cell surface proteins: Mesothelin, EpCam, CD19, CD20, CD38, CD3, TIM-3, CEA, cMet, ICAM1, ICAM3, MadCam, a4b7, CD7, CD4, CD138, integrins such as αVβ6, α4β7, α4β1, αEβ7.

Angiogenesis factors and Growth factors: VEGF, Angiopoietin 2, HGF, PDGF, EGF, GM-CSF, HB-EGF, TGF

Immune checkpoint inhibitors or activators: PD-1, PD-L1, CTLA4, CD28, B7-1, B7-2, ICOS, ICOSL, B7-H3, B7-H4, LAG3, KIR, 4-1BB, OX40, CD27, CD40L, TIM3, A2aR

Circulating proteins: TNFa, IL23, IL12, IL33, IL4, IL13, IL5, IL6, IL4, IFNg, IL17, RANKL, Bace1, alpha Synuclein, Tau, amyloid.

Hence are particularly foreseen
- A polypeptide comprising a variant as disclosed above, binding to HSA and an antibody binding to IL-17 or IL-17R (such as ixekizumab or brodalumab) for the treatment of autoimmune diseases such as Psoriasis
- A polypeptide comprising a variant as disclosed above, binding to HSA and an antibody binding to PD-L1 (such as atezolizumab, avelumab or durvalumab) for the treatment of cancers such as kidney, lung, breast, pancreatic cancers and various types of blood cancers such as multiple myeloma, lymphoma.
- A polypeptide comprising a variant as disclosed above, binding to HSA and an antibody binding to EGFR (such as cetuximab, panitumumab, zalutumumab, nimotuzumab, and matuzumab) to treat solid tumors.

Alternatively, the polypeptide may contain a variant as disclosed above, and a biologically active molecule, such as an erythropoietin, an interferon, a lysosomal disorder enzyme or etanercept.

In another embodiment, the variant of the OB-fold domain (in particular the variant of a protein of the Sac7d family) is conjugated to an organic molecule. This may be done by any method known in the art. In particular, one can chemically link the molecule to the protein. As a molecule, one can cite antiproliferative (cytotoxic and cytostatic agents) include cytotoxic compounds (e.g., broad spectrum), angiogenesis inhibitors, cell cycle progression inhibitors, PBK/m-TOR/AKT pathway inhibitors, MAPK signaling pathway inhibitors, kinase inhibitors, protein chaperones inhibitors, HDAC inhibitors, PARP inhibitors, Wnt/Hedgehog signaling pathway inhibitors, RNA polymerase inhibitors and proteasome inhibitors. One can also use anti-inflammatory molecules.

One can cite in particular DNA-binding or alkylating drugs such as anthracyclines (doxorubicin, epirubicin, idarubicin, daunorubicin) and its analogs, alkylating agents, such as calicheamicins, dactinomycines, mitromycines, pyrrolobenzodiazepines, and the like. One can also cite cell cycle progression inhibitors such as CDK inhibitors, Rho-kinase inhibitors, checkpoint kinase inhibitors, aurora kinase inhibitors, PLK inhibitors, and KSP inhibitors. One can also cite thalidomide and its derivatives lenalidomide and pomalidomide. In order for treating inflammation disorders, one can also use cyclooxygenase-2 inhibitors, 5-lipoxygenase inhibitors, quercetin and/or resveratrol as molecules conjugated to the polypeptide comprising the variant.

Hence are particularly foreseen
- A polypeptide comprising a variant as disclosed above, binding to HSA and a Janus Kinase inhibitor for the treatment of inflammatory diseases, in particular Crohns disease
- A polypeptide comprising a variant as disclosed above, binding to HSA and doxorubicin for the treatment of solid tumors such as pancreatic, ovarian, lung, kidney, liver, breast cancers.

Interesting and preferred molecules are also disclosed above.

### Use of the variants

The variants can be used in particular in therapeutic methods, especially for treating cancer, metabolic diseases (such as diabetes), neurologic disease, inflammatory and autoimmune diseases (such as rheumatoid arthritis, psoriasis, lupus or multiple sclerosis), or enzyme deficiency in particular in the brain, using biomolecules having activity for treatment of such diseases.

The invention thus relates to methods of treatment of cancer, metabolic diseases (such as diabetes), neurologic disease, inflammatory and autoimmune diseases (such as rheumatoid arthritis, psoriasis, lupus or multiple sclerosis), or enzyme deficiency in particular in the brain, comprising the administration of a therapeutic amount of a variant of an OB-fold herein disclosed (in particular a variant of a protein of the Sac7d family), fused to a molecule (polypeptide or other molecules with therapeutic effect against the disease to be treated) to a subject in need thereof.

The term "therapeutic amount" or "effective amount", as used herein, is the amount sufficient to effect beneficial or desired results, such as clinical results, and an "effective amount" depends upon the context in which it is being applied. An effective amount is an amount that provides therapeutic improvement while minimizing side or adverse effect. For cancer, therapeutic improvement may be regression of tumor size in solid tumor, improvement in life quality of the subject, improvement of the efficacy of a combined treatment.

One can administer the variant by any method of the art.

In particular, one can inject the variant. In another embodiment, one can apply the variant topically (either on the skin or on the eye of the patient) as disclosed in WO 2014/173899. In another embodiment, one can administer the variant orally, as disclosed in WO 2016/062874.

The variants or polypeptides containing the variants can also be used in diagnostic methods. In particular, such variants or polypeptide may be linked to any marker known in the art and used in imagery methods.

### DESCRIPTION OF THE FIGURES

Figure 1: alignment of proteins of the Sac7d family.
Figure 2: Binding kinetics profiles and values of B11 (panel A) and F06 (panel B) on human serum albumin (HSA) obtained by biolayer interferometry. B11 and F06 were run on streptavidin biosensors loaded with biotinylated HSA over a concentration range from 15.6 to 1000 nM. Association and dissociation were run for 180 sec each.
Figure 3: percentage of remaining fluorescein in serum after a single i.v. injection in mice either as a form of a conjugate with an anti-albumin Nanofitin (blue, n=3) or not (red). Data for the fluorescein alone were extracted from (Chahalet al., Invest. Ophthalmol. Vis. Sci. 26(1985)764-768) and are shown here as reference.
Figure 4: function of the densitometry versus the time of a anti-albumin variant fused with a protein partner.
Figure 5: ELISA signal of the Nanofitins B11 (panel A) and F06 (panel B) on the different domains of the human serum albumin.

### EXAMPLES

### Example 1. Characterization of variants of Sac7d binding to albumin

The affinity of the Nanofitins B11 (SEQ ID NO: 58) and F06 (SEQ ID NO: 65 with L56M) for the human serum albumin (HSA) was investigated by biolayer interferometry (octet red96) and is shown on Figure 2. Biotinylated HSA was immobilized on streptavidin biosensors and association (180 sec) and dissociation (180 sec) of the Nanofitins were investigated over a concentration range from 15.6 nM to 1000 nM. In these conditions, affinities of 0.1 and 8 nM were found respectively for B11 and F06. Similar affinities were obtained at pH 7.4 and pH 6.

**Table 10: Affinity and binding kinetic parameters of B11 and F06 measured by biolayer interferometry experiments.**

| Sample ID | KD (M) | ka (1M/s) | kis (1/s) | Full R^2 |
|---|---|---|---|---|
| B11 | 1.17E-10 | 2.31 E+05 | 2.71 E-05 | 0.9967 |
| F06 | 8.38E-09 | 3.63E+04 | 3.04E-04 | 0.9995 |

Using similar conditions, the ability of the two Nanofitins to bind on Mouse (MSA), Rat (RSA), Dog (DSA), Cynomolgus monkey (CSA), Porcine (PSA), Bovine (BSA) and Rabbit (RabSA) serum albumins was also investigated. Cross reactivity was found on all the albumins from the different species explored but the rabbit serum albumin.

These results were repeated with point mutations, namely D16E, N37Q and M57L, either individually or simultaneously, for both Nanofitins without noticeable change.

**Table 11: Table showing the cross reactivity pattern of B11 and F06 on albumins from different species. N.D. stands for not done. Increasing "3" symbol refers to an increasing strength of binding. "-" symbol refers to an absence of detectable binding.**

| | HSA | MSA | RSA | DSA | CSA | PSA | BSA | RabSa |
|---|---|---|---|---|---|---|---|---|
| B11 | ++++ | ++ | ++ | N.D. | ++++ | N.D. | N.D. | - |
| F06 | +++ | ++ | ++ | ++ | +++ | ++ | ++ | - |

### Example 2. The variants increase the half-life of a small molecule

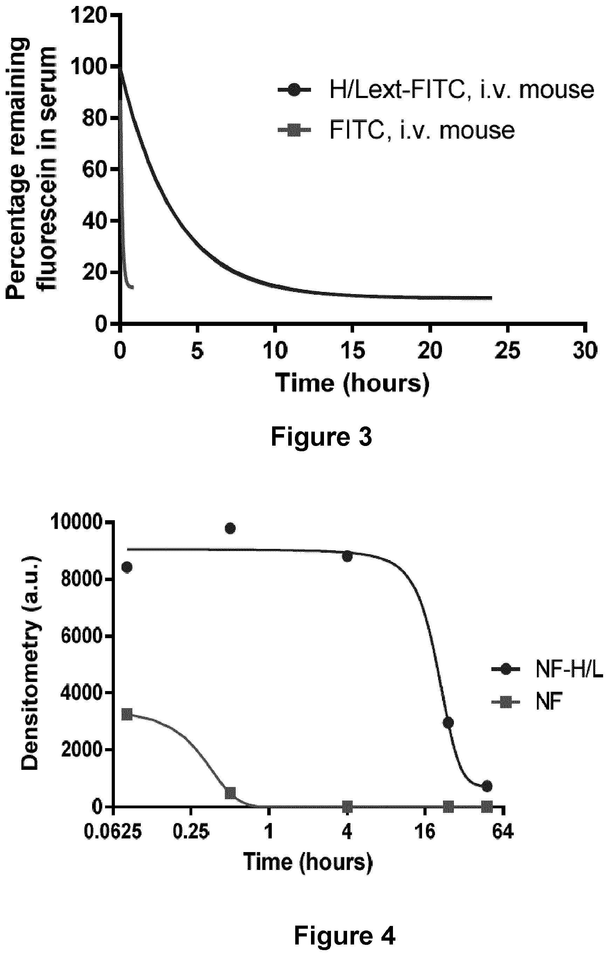

To exemplify the ability of the anti-albumin Nanofitin at extending the serum half-life (t_{1/2}) of a cargo small molecule, we used the fluorescein as a model. Fluorescein is a small molecule (332 Da) that is extensively used as a fluorescent tracer. Its biodisponibility in mouse serum after a single i.v. injection has been already studied and reported. Analysis of the data from the literature (Chahalet al., Invest. Ophthalmol. Vis. Sci. 26(1985)764-768) suggests a t_{1/2} of about 8 min. Fluorescein, under its isothiocyanate derivative (FITC), has been conjugated to the anti-albumin Nanofitin. After a single bolus i.v. injection in mice (20 mg/kg, 3 mice per time points) of the anti-albumin Nanofitin-Fluorescein conjugate, the presence of the fluorescein in serum could be tracked down by monitoring the fluorescence (excitation: 488 nm and emission: 520 nm) of the serum samples collected at intervals of time (0, 1, 4, 6 and 24 hours), as shown in Figure 3. The resulting t_{1/2} in mouse serum of the fluorescein when conjugated to the anti-albumin was calculated to be of 174 min.

### Example 3. Increasing the half-life of a protein partner

To exemplify the ability of the anti-albumin Nanofitin at extending the serum half-life (t_{1/2}) of a cargo protein molecule, we used another Nanofitin targeting TNFalpha as a model. Nanofitin are small protein (7 kDa) that are naturally rapidly excreted from the systemic circulation due to their size that is below the glomerular filtration cut off (50 kDa) of the kidney and the intrinsic absence of recycling. The anti-TNFalpha Nanofitin and a chimeric construction consisting in the genetic fusion of the anti-TNFalpha Nanofitin with an anti-albumin Nanofitin were injected in mice as a single bolus injection. Bleeding was performed at different intervals of time (5 min, 0.5, 4, 24 and 48 hours), and monitoring of the presence of the Nanofitin products in the serum was performed by western blot using and anti-histag antibody for the revelation. The data was plot as a function of the densitometry versus the time (Figure 4). While the anti-TNFalpha Nanofitin showed a rapid clearance with a t_{1/2} of about 10 to 15 min, the fusion with the anti-albumin Nanofitin resulted in a prolonged retention in the serum with a t_{1/2} above 16 hours in mice.

### Example 4. Domain that is targeted by the anti-albumin Nanofitin

The albumin is described as having three domains, dubbed domain I, II and III. Interaction of the albumin with the FcRn receptor involves domain I and III. Domain I (aa1-197), domain II (aa189-385), and domain III (aa381-585) and Domain I-II (aa1-385) were obtained from Albumin Bioscience. Interaction of the anti-albumin Nanofitins with the different domains was investigated by biolayer inetrferometry according to the following procedure. Histagged Nanofitins B11 and F06 were immobilized on Ni:NTA biosensors at 1 nm. The sensors were then allowed to equilibrate for 60 sec in protein free blocking buffer (Thermo Scientific) supplemented with 0.002 % of tween20. Association of the different albumin domains (250 nM in protein free blocking buffer supplemented with 0.002 % of tween20) was monitored for 180 sec. The response at 180 sec was then recorded and used to reflect the binding ability of B11 and F06 Nanofitins for the different albumin domains.

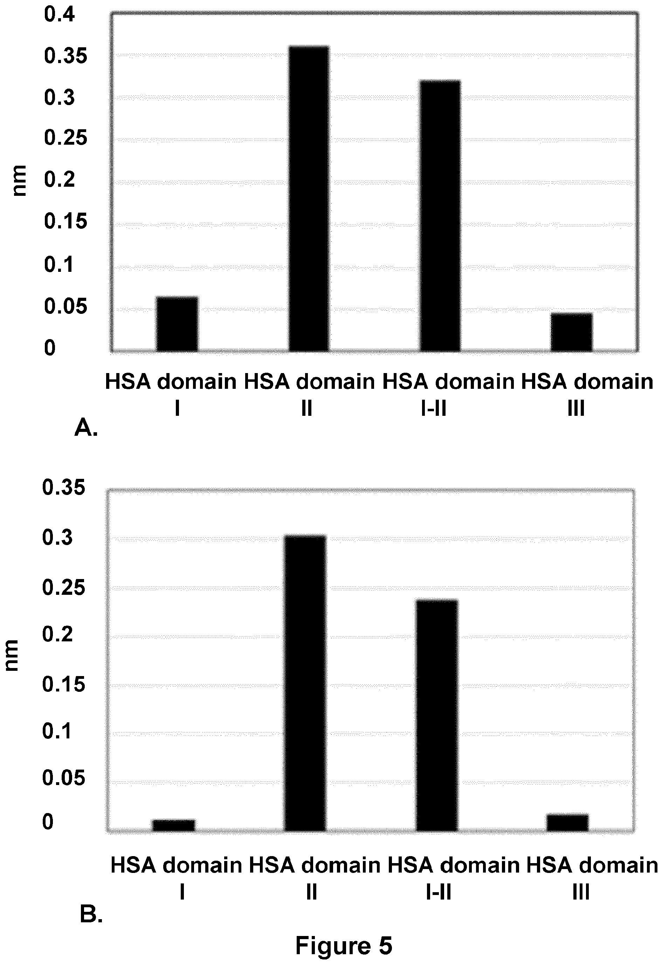

Both Nanofitins were found to interact only with the domain I-II and II, which demonstrate that they are targeting the domain II of the albumin (Figure 5).

This was further demonstrated by a competition experiment with an albumin binding domain G148-GA3 (He et al.; Protein Science; 16(2007)1490-1494). The interaction of the two anti-albumin Nanofitins is inhibited by the presence of G148-GA3. It has been shown that G148-GA3 binds the domain II of the HSA (Lejon et al.; J. Biol. Chem.; 279(2004)42924-42928). The domain II of the HSA stands away from the interaction site of the albumin with the FcRn. By targeting the domain II of the HSA, anti-albumin Nanofitin can engage the albumin without impairing its binding with the FcRn, allowing the Nanofitins to benefit from its recycling mechanism for extending its serum half-life. This is further true considering that the affinity of the anti-albumin Nanofitins remains similar at both pH 7.4 and pH 6, allowing them to remain engaged on their target during the recycling process.

## Claims

1. A protein comprising a variant of a member of the Sac7d family binding to human and mouse serum albumin, wherein the variant comprises from 4 to 20 mutated residues in the interface of binding of the member of the Sac7d family to its natural ligand, and wherein said variants comprises K22W, W24L, T33K, R42Y mutations with the numbering corresponding to the position in the Sac7d sequence SEQ ID NO: 1.

2. The polypeptide of claim 1, further comprising at least one mutation selected from D16E, N37Q and M57L, with the numbering corresponding to the position in the Sac7d sequence SEQ ID NO: 1.

3. The polypeptide of claim 1 or 2, wherein the mutated residues in the interface of binding of the member of the Sac7d family to its natural ligand are selected from the group consisting of V2, K3, K5, K7, Y8, K9, G10, E14, T17, K21, K22, W24, V26, G27, K28, M29, S31, T33, D36, N37, G38, K39, T40, A44, S46, E47, K48, D49, A50 and P51 of Sac7d.

4. The polypeptide of any one of claim 1 to 3, further comprising at least one mutation selected from Y8F, Y8P, A44F, A44V, with the numbering corresponding to the position in the Sac7d sequence SEQ ID NO: 1.

5. The polypeptide of any one of claim 1 to 4, which is selected from the group consisting of Sac7d from *Sulfolobus acidocaldarius,* Sac7e from *Sulfolobus acidocaldarius,* SSo7d from *Sulfolobus solfataricus,* Ssh7b from *Sulfolobus shibatae,* Ssh7a from *Sulfolobus shibatae,* DBP7 from *Sulfolobus tokodaii,* Sis7a from *Sulfolobus islandicus,* Mse7 from *Metallosphaera sedula,* Mcu7 from *Metallosphaera cuprina,* Aho7a from *Acidianus hospitalis,* Aho7b from *Acidianus hospitalis,* Aho7c from *Acidianus hospitalis* and Sto7 from *Sulfurisphaera tokodaii.*

6. The polypeptide of any one of claims 1 to 5, which comprises SEQ ID NO: 38 or amino acids 1-54 of SEQ ID NO: 38.

7. The polypeptide of claim 6, which comprises a sequence selected from SEQ ID NO: 48, SEQ ID NO: 51, SEQ ID NO: 55, SEQ ID NO: 58 SEQ ID NO: 62, SEQ ID NO: 65, and amino acids 1-57 of these sequences.

8. The polypeptide of claim 6, which comprises a sequence selected from SEQ ID NO: 69, SEQ ID NO: 72, SEQ ID NO: 76, SEQ ID NO: 79 SEQ ID NO: 83, SEQ ID NO: 86, and amino acids 1-55 of these sequences.

9. The polypeptide of any one of claims 1 to 8 which consists in the variant of a member of the Sac7d family binding to human and mouse serum albumin.

10. The polypeptide of any one of claims 1 to 8, wherein the variant of a member of the Sac7d family binding to human and mouse serum albumin is conjugated to an organic molecule.

11. The polypeptide of any one of claims 1 to 8, wherein the variant of a member of the Sac7d family binding to human and mouse serum albumin is conjugated to another polypeptide, in particular another variant of a protein of the Sac7d family.

12. A nucleic acid molecule coding for the polypeptide of any one of claims 1 to 9, and 11.

13. A pharmaceutical composition comprising the polypeptide of any one of claims 1-11, or the nucleic acid of claim 12, and a pharmaceutically acceptable carrier

14. A method for producing the polypeptide of any one of claims 1 to 9 and 11 to 12, comprising the steps consisting of
(a) Culturing a cell culture wherein the cells have been transformed by the nucleic acid of claim 12,
And
(b) Recovering the polypeptide.

15. The polypeptide of any one of claims 10 or 11 as a medicament.


SEQUENCE LISTING

<110>  AFFILOGIC

<120>  COMPOUNDS AND METHODS FOR EXTENDING HALF LIFE OF BIOMOLECULES

<130>  BRV 195 - EP PRIO

<160>  87

<170>  PatentIn version 3.5

<210>  1
<211>  66
<212>  PRT
<213>  Sulfolobus acidocaldarius

<400>  1

Met Val Lys Val Lys Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Asp Met Leu Ala Arg Ala Glu Arg Glu
    50                  55                  60

Lys Lys
65

<210>  2
<211>  64
<212>  PRT
<213>  Sulfolobus solfataricus

<400>  2

Met Ala Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  3

<211>  65
<212>  PRT
<213>  Sulfolobus acidocaldarius

<400>  3

Met Ala Lys Val Arg Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Met Asp Met Leu Ala Arg Ala Glu Lys Lys
    50                  55                  60

Lys
65

<210>  4
<211>  64
<212>  PRT
<213>  Sulfolobus shibatae

<400>  4

Met Val Thr Val Lys Phe Lys Tyr Lys Gly Glu Glu Lys Glu Val Asp
1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  5
<211>  64
<212>  PRT
<213>  Sulfolobus shibatae

<400>  5

Met Ala Thr Val Lys Phe Lys Tyr Lys Gly Glu Glu Lys Gln Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  6
<211>  64
<212>  PRT
<213>  Sulfolobus tokodaii

<400>  6

Met Val Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Gln Met Leu Glu Lys Ser Gly Lys Lys
    50                  55                  60

<210>  7
<211>  64
<212>  PRT
<213>  Sulfolobus islandicus

<400>  7

Met Val Thr Val Lys Phe Lys Tyr Lys Gly Glu Glu Lys Gln Val Asp
1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  8
<211>  64
<212>  PRT
<213>  Sulfolobus islandicus

<400>  8

Met Thr Thr Val Lys Phe Lys Tyr Lys Gly Glu Glu Lys Gln Val Asp

1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  9
<211>  64
<212>  PRT
<213>  Sulfolobus islandicus

<400>  9

Met Thr Thr Val Lys Phe Lys Tyr Lys Gly Glu Glu Lys Gln Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  10
<211>  62
<212>  PRT
<213>  Metallosphaera sedula.

<400>  10

Met Ala Thr Lys Ile Lys Phe Lys Tyr Lys Gly Gln Asp Leu Glu Val
1               5                   10                  15

Asp Ile Ser Lys Val Lys Lys Val Trp Lys Val Gly Lys Met Val Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Asn Met Ile Gly Lys Lys
    50                  55                  60

<210>  11
<211>  62

<212>  PRT
<213>  Metallosphaera cuprina

<400>  11

Met Ala Thr Lys Ile Lys Phe Lys Tyr Lys Gly Gln Asp Leu Glu Val
1               5                   10                  15

Asp Ile Ser Lys Val Lys Lys Val Trp Lys Val Gly Lys Met Val Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Ser Met Ile Gly Lys Lys
    50                  55                  60

<210>  12
<211>  61
<212>  PRT
<213>  Acidianus hospitalis

<400>  12

Met Thr Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Glu Lys Leu Glu Lys Lys
    50                  55                  60

<210>  13
<211>  62
<212>  PRT
<213>  Acidianus hospitalis

<400>  13

Met Ala Thr Lys Val Lys Phe Lys Tyr Lys Gly Glu Glu Lys Glu Val
1               5                   10                  15

Asp Ile Ser Lys Ile Lys Lys Val Trp Arg Val Gly Lys Met Ile Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Asp Lys Leu Glu Lys Lys
    50                  55                  60

<210>  14
<211>  60
<212>  PRT
<213>  Acidianus hospitalis

<400>  14

Met Ala Thr Lys Val Lys Phe Lys Tyr Lys Gly Glu Glu Lys Glu Val
1               5                   10                  15

Asp Ile Ser Lys Ile Lys Lys Val Trp Arg Val Gly Lys Met Ile Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Glu Lys Leu Lys
    50                  55                  60

<210>  15
<211>  64
<212>  PRT
<213>  Sulfurisphaera tokodaii

<400>  15

Met Val Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Gln Met Leu Glu Lys Ser Gly Lys Lys
    50                  55                  60

<210>  16
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  16

Met Xaa Xaa Xaa Val Xaa Phe Lys Tyr Lys Gly Glu Glu Lys Xaa Val
1               5                   10                  15

Asp Xaa Ser Lys Ile Lys Lys Val Trp Arg Val Gly Lys Met Xaa Ser
            20                  25                  30

Phe Thr Tyr Asp Xaa Xaa Xaa Gly Lys Thr Gly Arg Gly Ala Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  17
<211>  68
<212>  PRT
<213>  Artificial Sequence

<220>
<223>  Sac7d Family consensus sequence Albumin-binding protein

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is W,F,Y,V,I or L

<220>
<221>  VARIANT
<222>  (9)..(9)
<223>  Xaa is P or F

<220>
<221>  VARIANT
<222>  (10)..(10)
<223>  Xaa is R,K,S,T,Q or N

<220>
<221>  VARIANT
<222>  (15)..(15)

<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (46)..(46)
<223>  Xaa is V or F

<220>
<221>  VARIANT
<222>  (48)..(48)
<223>  Xaa is any amino acid

<220>
<221>  VARIANT
<222>  (57)..(57)
<223>  Xaa is M or L

<220>

<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  17

Met Xaa Xaa Xaa Val Xaa Phe Xaa Xaa Xaa Gly Glu Glu Lys Xaa Val
1               5                   10                  15

Xaa Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Xaa Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  18
<211>  68
<212>  PRT
<213>  Artificial Sequence

<220>
<223>  Sac7d Family consensus sequence F06

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT

<222>  (6)..(6)
<223>  Xaa is R or K

<220>
<221>  VARIANT
<222>  (8)..(8)
<223>  Xaa is W,F,Y,V,I or L

<220>
<221>  VARIANT
<222>  (10)..(10)
<223>  Xaa is R,K,S,T,Q or N

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  18

Met Xaa Xaa Xaa Val Xaa Phe Xaa Phe Xaa Gly Glu Glu Lys Xaa Val
1               5                   10                  15

Xaa Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Phe Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  19
<211>  68
<212>  PRT
<213>  Artificial Sequence

<220>
<223>  Sac7d Family consensus sequence F06

<220>
<221>  VARIANT
<222>  (2)..(2)
<223>  Xaa is V, A or T

<220>

<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  19

Met Xaa Xaa Xaa Val Xaa Phe Val Phe Ser Gly Glu Glu Lys Xaa Val
1               5                   10                  15

Xaa Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Phe Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  20
<211>  68
<212>  PRT
<213>  Artificial Sequence

<220>
<223>  Sac7d Family consensus sequence F06

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  20

Met Xaa Xaa Xaa Val Xaa Phe Val Phe Ser Gly Glu Glu Lys Xaa Val
1               5                   10                  15

Xaa Xaa Ser Lys Ile Lys Trp Val Leu Arg Trp Gly Lys Ala Xaa Gly
            20                  25                  30

Phe Lys Tyr Asp Xaa Xaa Xaa Gly Lys Ile Gly Tyr Gly Phe Val Ala
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  21
<211>  68
<212>  PRT
<213>  Artificial Sequence

<220>
<223>  Sac7d Family consensus sequence B11

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is W,F,Y,V,I or L

<220>
<221>  VARIANT
<222>  (10)..(10)
<223>  Xaa is R,K,S,T,Q or N

<220>
<221>  VARIANT
<222>  (15)..(15)
<223>  Xaa is E or Q

<220>
<221> VARIANT
<222> (17)..(17)
<223> Xaa is D or E

<220>
<221> VARIANT
<222> (18)..(18)
<223> Xaa is T or I

<220>
<221> VARIANT
<222> (22)..(22)
<223> Xaa is any amino acid

<220>
<221> VARIANT
<222> (27)..(27)
<223> Xaa is any amino acid

<220>
<221> VARIANT
<222> (30)..(30)
<223> Xaa is any amino acid

<220>
<221> VARIANT
<222> (31)..(31)
<223> Xaa is V or I

<220>
<221> VARIANT
<222> (32)..(32)
<223> Xaa is any amino acid

<220>
<221> VARIANT
<222> (37)..(39)
<223> Xaa Xaa Xaa is EGG or DN-

<220>
<221> VARIANT
<222> (42)..(42)
<223> Xaa is any amino acid

<220>
<221> VARIANT
<222> (48)..(48)
<223> Xaa is any amino acid

<220>
<221> VARIANT
<222> (57)..(57)
<223> Xaa is M or L

<220>
<221> VARIANT
<222> (58)..(58)
<223> Xaa is Q, D or E

<220>
<221> VARIANT

<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
       ARAEREKK, ARAERE-K, ARAERE--, ARA-EKKK, E-----KK or EKS--GKK

<400>  21

Met Xaa Xaa Xaa Val Xaa Phe Xaa Phe Xaa Gly Glu Glu Lys Xaa Val
1               5                   10                  15

Xaa Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Phe Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  22
<211>  68
<212>  PRT
<213>  Artificial Sequence

<220>
<223>  Sac7d Family consensus sequence B11

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)

<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>

<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
       ARAEREKK, ARAERE-K, ARAERE--, ARA-EKKK, E-----KK or EKS--GKK

<400>  22

Met Xaa Xaa Xaa Val Xaa Phe Val Phe Ser Gly Glu Glu Lys Xaa Val
1               5                   10                  15

Xaa Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Phe Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  23
<211>  68
<212>  PRT
<213>  Artificial Sequence

<220>
<223>  Sac7d Family consensus sequence B11

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT

<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  23

Met Xaa Xaa Xaa Val Xaa Phe Val Phe Ser Gly Glu Glu Lys Xaa Val
1               5                   10                  15

Xaa Xaa Ser Lys Ile Lys Trp Val Leu Arg Trp Gly Lys Ala Xaa Gly
            20                  25                  30

Phe Lys Tyr Asp Xaa Xaa Xaa Gly Lys Ile Gly Tyr Gly Phe Val Ala
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa

65

<210>  24
<211>  68
<212>  PRT
<213>  Artificial Sequence

<220>
<223>  Sac7d Family consensus sequence Albumin-binding protein

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is W,F,Y,V,I or,L

<220>
<221>  VARIANT
<222>  (9)..(9)
<223>  Xaa is P or F

<220>
<221>  VARIANT
<222>  (10)..(10)
<223>  Xaa is R,K,S,T,Q or N

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>

<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (46)..(46)
<223>  Xaa is V or F

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,

ARAEREKK, ARAERE-K, ARAERE--, ARA-EKKK, E-----KK or EKS--GKK

<400> 24

Met Xaa Xaa Xaa Val Xaa Phe Xaa Xaa Xaa Gly Glu Glu Lys Xaa Val
1               5                   10                  15

Asp Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Xaa Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210> 25
<211> 68
<212> PRT
<213> Artificial Sequence

<220>
<223> Sac7d Family consensus sequence F06

<220>
<221> VARIANT
<222> (2)..(2)
<223> Xaa is V, A or T

<220>
<221> VARIANT
<222> (3)..(3)
<223> Xaa is T or K

<220>
<221> VARIANT
<222> (4)..(4)
<223> Xaa is - or K

<220>
<221> VARIANT
<222> (6)..(6)
<223> Xaa is R or K

<220>
<221> VARIANT
<222> (8)..(8)
<223> Xaa is W,F,Y,V,I or L

<220>
<221> VARIANT
<222> (10)..(10)
<223> Xaa is R,K,S,T,Q or N

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)

<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  25

Met Xaa Xaa Xaa Val Xaa Phe Xaa Phe Xaa Gly Glu Glu Lys Xaa Val
1               5                   10                  15

Asp Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Phe Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  26
<211>  68
<212>  PRT
<213>  Artificial Sequence

<220>
<223>  Sac7d Family consensus sequence F06

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT

<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
       ARAEREKK, ARAERE-K, ARAERE--, ARA-EKKK, E-----KK or EKS--GKK

<400>  26

Met Xaa Xaa Xaa Val Xaa Phe Val Phe Ser Gly Glu Glu Lys Xaa Val
1               5                   10                  15

Asp Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Phe Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  27
<211>  68
<212>  PRT
<213>  Artificial Sequence

<220>
<223>  Sac7d Family consensus sequence F06

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)

<223>  Xaa is R or K

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  27

Met Xaa Xaa Xaa Val Xaa Phe Val Phe Ser Gly Glu Glu Lys Xaa Val
1               5                   10                  15

Asp Xaa Ser Lys Ile Lys Trp Val Leu Arg Trp Gly Lys Ala Xaa Gly
            20                  25                  30

Phe Lys Tyr Asp Xaa Xaa Xaa Gly Lys Ile Gly Tyr Gly Phe Val Ala
        35                  40                  45

Glu Lys Asp Ala Pro Lys Glu Leu Xaa Xaa Xaa Leu Xaa Xaa Xaa Xaa

50                  55                  60

Xaa Xaa Xaa Xaa
65

<210>  28
<211>  68
<212>  PRT
<213>  Artificial Sequence

<220>
<223>  Sac7d Family consensus sequence B11

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is W,F,Y,V,I or L

<220>
<221>  VARIANT
<222>  (10)..(10)
<223>  Xaa is R,K,S,T,Q or N

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT

<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
       ARAEREKK, ARAERE-K, ARAERE--, ARA-EKKK, E-----KK or EKS--GKK

<400>  28

Met Xaa Xaa Xaa Val Xaa Phe Xaa Pro Xaa Gly Glu Glu Lys Xaa Val
1               5                   10                  15

Asp Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Val Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  29
<211>  68
<212>  PRT
<213>  Artificial Sequence

<220>
<223>  Sac7d Family consensus sequence B11

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>

<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  29

Met Xaa Xaa Xaa Val Xaa Phe Trp Pro Arg Gly Glu Glu Lys Xaa Val

1                5                   10                  15

Asp Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Val Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  30
<211>  68
<212>  PRT
<213>  Artificial Sequence

<220>
<223>  Sac7d Family consensus sequence B11

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  30

Met Xaa Xaa Xaa Val Xaa Phe Trp Pro Arg Gly Glu Glu Lys Xaa Val
1               5                   10                  15

Asp Xaa Ser Lys Ile Ala Trp Val Leu Arg Ala Asp Lys Thr Xaa Met
            20                  25                  30

Phe Lys Tyr Asp Xaa Xaa Xaa Gly Lys Lys Gly Tyr Gly Val Val Leu
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  31
<211>  68
<212>  PRT
<213>  Artificial Sequence

<220>
<223>  Sac7d Family consensus sequence Albumin-binding protein

<220>

<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is W,F,Y,V,I or L

<220>
<221>  VARIANT
<222>  (9)..(9)
<223>  Xaa is P or F

<220>
<221>  VARIANT
<222>  (10)..(10)
<223>  Xaa is R,K,S,T,Q or N

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (46)..(46)
<223>  Xaa is V or F

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  31

Met Xaa Xaa Xaa Val Xaa Phe Xaa Xaa Xaa Gly Glu Glu Lys Xaa Val
1               5                   10                  15

Glu Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Xaa Val Xaa

35                    40                    45

Glu Lys Asp Ala Pro Lys Glu Leu Xaa Xaa Xaa Leu Xaa Xaa Xaa Xaa
    50                    55                    60

Xaa Xaa Xaa Xaa
65

<210>  32
<211>  68
<212>  PRT
<213>  Artificial Sequence

<220>
<223>  Sac7d Family consensus sequence F06

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is W,F,Y,V,I or L

<220>
<221>  VARIANT
<222>  (10)..(10)
<223>  Xaa is R,K,S,T,Q or N

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)

<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  32

Met Xaa Xaa Xaa Val Xaa Phe Xaa Phe Xaa Gly Glu Glu Lys Xaa Val
1               5                   10                  15

Glu Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Phe Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  33
<211>  68
<212>  PRT
<213>  Artificial Sequence

<220>
<223>  Sac7d Family consensus sequence F06

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT

<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  33

Met Xaa Xaa Xaa Val Xaa Phe Val Phe Ser Gly Glu Glu Lys Xaa Val
1               5                   10                  15

Glu Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Phe Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  34
<211>  68
<212>  PRT
<213>  Artificial Sequence

<220>
<223>  Sac7d Family consensus sequence F06

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is D or E

<220>

<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  34

Met Xaa Xaa Xaa Val Xaa Phe Val Phe Ser Gly Glu Glu Lys Xaa Val
1               5                   10                  15

Glu Xaa Ser Lys Ile Lys Trp Val Leu Arg Trp Gly Lys Ala Xaa Gly
            20                  25                  30

Phe Lys Tyr Asp Xaa Xaa Xaa Gly Lys Ile Gly Tyr Gly Phe Val Ala
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  35
<211>  68
<212>  PRT
<213>  Artificial Sequence

<220>

<223>  Sac7d Family consensus sequence B11

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is W,F,Y,V,I or L

<220>
<221>  VARIANT
<222>  (10)..(10)
<223>  Xaa is R,K,S,T,Q or N

<220>
<221>  VARIANT
<222>  (15)..(15)
<223>  Xaa is E or Q

<220>
<221>  VARIANT
<222>  (17)..(17)
<223>  Xaa is D or E

<220>
<221>  VARIANT
<222>  (18)..(18)
<223>  Xaa is T or I

<220>
<221>  VARIANT
<222>  (22)..(22)
<223>  Xaa is any amino acid

<220>
<221>  VARIANT
<222>  (27)..(27)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  35

Met Xaa Xaa Xaa Val Xaa Phe Xaa Pro Xaa Gly Glu Glu Lys Xaa Val
1               5                   10                  15

Glu Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Val Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210> 36
<211> 68
<212> PRT
<213> Artificial Sequence

<220>
<223> Sac7d Family consensus sequence B11

<220>
<221> VARIANT
<222> (2)..(2)
<223> Xaa is V, A or T

<220>
<221> VARIANT
<222> (3)..(3)
<223> Xaa is T or K

<220>
<221> VARIANT
<222> (4)..(4)
<223> Xaa is - or K

<220>
<221> VARIANT
<222> (6)..(6)
<223> Xaa is R or K

<220>
<221> VARIANT
<222> (15)..(15)
<223> Xaa is E or Q

<220>
<221> VARIANT
<222> (17)..(17)
<223> Xaa is D or E

<220>
<221> VARIANT
<222> (18)..(18)
<223> Xaa is T or I

<220>
<221> VARIANT
<222> (22)..(22)
<223> Xaa is any amino acid

<220>
<221> VARIANT
<222> (27)..(27)
<223> Xaa is any amino acid

<220>
<221> VARIANT
<222> (30)..(30)
<223> Xaa is any amino acid

<220>
<221>  VARIANT
<222>  (31)..(31)
<223>  Xaa is V or I

<220>
<221>  VARIANT
<222>  (32)..(32)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  36

Met Xaa Xaa Xaa Val Xaa Phe Trp Pro Arg Gly Glu Glu Lys Xaa Val
1               5                   10                  15

Glu Xaa Ser Lys Ile Xaa Trp Val Leu Arg Xaa Gly Lys Xaa Xaa Xaa
            20                  25                  30

Phe Lys Tyr Asp Xaa Xaa Xaa Gly Lys Xaa Gly Tyr Gly Val Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210> 37
<211> 68
<212> PRT
<213> Artificial Sequence

<220>
<223> Sac7d Family consensus sequence B11

<220>
<221> VARIANT
<222> (2)..(2)
<223> Xaa is V, A or T

<220>
<221> VARIANT
<222> (3)..(3)
<223> Xaa is T or K

<220>
<221> VARIANT
<222> (4)..(4)
<223> Xaa is - or K

<220>
<221> VARIANT
<222> (6)..(6)
<223> Xaa is R or K

<220>
<221> VARIANT
<222> (15)..(15)
<223> Xaa is E or Q

<220>
<221> VARIANT
<222> (17)..(17)
<223> Xaa is D or E

<220>
<221> VARIANT
<222> (18)..(18)
<223> Xaa is T or I

<220>
<221> VARIANT
<222> (31)..(31)
<223> Xaa is V or I

<220>
<221> VARIANT
<222> (37)..(39)
<223> Xaa Xaa Xaa is EGG or DN-

<220>
<221> VARIANT
<222> (57)..(57)

<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK or EKS--GKK

<400>  37

Met Xaa Xaa Xaa Val Xaa Phe Trp Pro Arg Gly Glu Glu Lys Xaa Val
1               5                   10                  15

Glu Xaa Ser Lys Ile Ala Trp Val Leu Arg Ala Asp Lys Thr Xaa Met
            20                  25                  30

Phe Lys Tyr Asp Xaa Xaa Xaa Gly Lys Lys Gly Tyr Gly Val Val Leu
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  38
<211>  64
<212>  PRT
<213>  Artificial Sequence

<220>
<223>  Sac7d/Aho7c consensus sequence Albumin-binding protein

<220>
<221>  VARIANT
<222>  (1)..(1)
<223>  Xaa is V or T

<220>
<221>  VARIANT
<222>  (6)..(6)
<223>  Xaa is W,F,Y,V,I or L

<220>
<221>  VARIANT
<222>  (7)..(7)
<223>  Xaa is P or F

<220>
<221>  VARIANT
<222>  (8)..(8)
<223>  Xaa is R,K,S,T,Q or N

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (43)..(43)
<223>  Xaa is V or F

<220>
<221>  VARIANT
<222>  (45)..(45)
<223>  Xaa is any amino acid

<220>
<221>  VARIANT

<222>  (55)..(64)
<223>  XaaXaaXaaXaaXaaXaaXaaXaaXaaXaa is DMLARAEREK or EKLK------

<400>  38

Xaa Lys Val Lys Phe Xaa Xaa Xaa Gly Glu Glu Lys Glu Val Xaa Xaa
1               5                   10                  15

Ser Lys Ile Xaa Trp Val Leu Arg Xaa Gly Lys Xaa Xaa Xaa Phe Lys
            20                  25                  30

Tyr Asp Asp Xaa Gly Lys Xaa Gly Tyr Gly Xaa Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  39
<211>  64
<212>  PRT
<213>  Artificial Sequence

<220>
<223>  Sac7d/Aho7c consensus sequence Albumin-binding protein F06

<220>
<221>  VARIANT
<222>  (1)..(1)
<223>  Xaa is V or T

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (20)..(20)
<223>  Xaa is any amino acid

<220>
<221>  VARIANT
<222>  (25)..(25)

<223>  Xaa is any amino acid

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)
<223>  Xaa is any amino acid

<220>
<221>  VARIANT
<222>  (55)..(64)
<223>  XaaXaaXaaXaaXaaXaaXaaXaaXaaXaa is DMLARAEREK or EKLK------

<400>  39

Xaa Lys Val Lys Phe Xaa Phe Xaa Gly Glu Glu Lys Glu Val Xaa Xaa
1               5                   10                  15

Ser Lys Ile Xaa Trp Val Leu Arg Xaa Gly Lys Xaa Xaa Xaa Phe Lys
            20                  25                  30

Tyr Asp Asp Xaa Gly Lys Xaa Gly Tyr Gly Phe Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  40
<211>  64
<212>  PRT
<213>  Artificial Sequence

<220>
<223>  Sac7d/Aho7c consensus sequence Albumin-binding protein F06

<220>
<221> VARIANT
<222> (1)..(1)
<223> Xaa is V or T

<220>
<221> VARIANT
<222> (15)..(15)
<223> Xaa is D or E

<220>
<221> VARIANT
<222> (16)..(16)
<223> Xaa is T or I

<220>
<221> VARIANT
<222> (20)..(20)
<223> Xaa is any amino acid

<220>
<221> VARIANT
<222> (25)..(25)
<223> Xaa is any amino acid

<220>
<221> VARIANT
<222> (28)..(28)
<223> Xaa is any amino acid

<220>
<221> VARIANT
<222> (29)..(29)
<223> Xaa is V or I

<220>
<221> VARIANT
<222> (30)..(30)
<223> Xaa is any amino acid

<220>
<221> VARIANT
<222> (36)..(36)
<223> Xaa is N or Q

<220>
<221> VARIANT
<222> (39)..(39)
<223> Xaa is any amino acid

<220>
<221> VARIANT
<222> (45)..(45)
<223> Xaa is any amino acid

<220>
<221> VARIANT
<222> (55)..(64)
<223> XaaXaaXaaXaaXaaXaaXaaXaaXaaXaa is DMLARAEREK or EKLK------

<400> 40

Xaa Lys Val Lys Phe Val Phe Ser Gly Glu Glu Lys Glu Val Xaa Xaa
1               5                   10                  15

Ser Lys Ile Xaa Trp Val Leu Arg Xaa Gly Lys Xaa Xaa Xaa Phe Lys
            20                  25                  30

Tyr Asp Asp Xaa Gly Lys Xaa Gly Tyr Gly Phe Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  41
<211>  64
<212>  PRT
<213>  Artificial Sequence

<220>
<223>  Sac7d/Aho7c consensus sequence Albumin-binding protein F06

<220>
<221>  VARIANT
<222>  (1)..(1)
<223>  Xaa is V or T

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (36)..(36)
<223>  Xaa is N or Q

<220>
<221>  VARIANT
<222>  (55)..(64)
<223>  XaaXaaXaaXaaXaaXaaXaaXaaXaaXaa is DMLARAEREK or EKLK------

<400>  41

Xaa Lys Val Lys Phe Val Phe Ser Gly Glu Glu Lys Glu Val Xaa Xaa
1               5                   10                  15

Ser Lys Ile Lys Trp Val Leu Arg Trp Gly Lys Ala Xaa Gly Phe Lys
            20                  25                  30

Tyr Asp Asp Xaa Gly Lys Ile Gly Tyr Gly Phe Val Ala Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  42
<211>  64
<212>  PRT
<213>  Artificial Sequence

<220>
<223>  Sac7d/Aho7c consensus sequence Albumin-binding protein B11

<220>
<221>  VARIANT
<222>  (1)..(1)
<223>  Xaa is V or T

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)
<223>  Xaa is any amino acid

<220>
<221>  VARIANT
<222>  (55)..(64)
<223>  XaaXaaXaaXaaXaaXaaXaaXaaXaaXaa is DMLARAEREK or EKLK------

<400>  42

Xaa Lys Val Lys Phe Xaa Pro Xaa Gly Glu Glu Lys Glu Val Xaa Xaa
1               5                   10                  15

Ser Lys Ile Xaa Trp Val Leu Arg Xaa Gly Lys Xaa Xaa Xaa Phe Lys
            20                  25                  30

Tyr Asp Asp Xaa Gly Lys Xaa Gly Tyr Gly Val Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  43
<211>  64
<212>  PRT
<213>  Artificial Sequence

<220>
<223>  Sac7d/Aho7c consensus sequence Albumin-binding protein B11

<220>
<221>  VARIANT
<222>  (1)..(1)
<223>  Xaa is V or T

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)
<223>  Xaa is any amino acid

<220>
<221>  VARIANT
<222>  (55)..(64)
<223>  XaaXaaXaaXaaXaaXaaXaaXaaXaaXaa is DMLARAEREK or EKLK------

<400>  43

Xaa Lys Val Lys Phe Trp Pro Arg Gly Glu Glu Lys Glu Val Xaa Xaa
1               5                   10                  15

Ser Lys Ile Xaa Trp Val Leu Arg Xaa Gly Lys Xaa Xaa Xaa Phe Lys
            20                  25                  30

Tyr Asp Asp Xaa Gly Lys Xaa Gly Tyr Gly Val Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  44
<211>  64
<212>  PRT
<213>  Artificial Sequence

<220>
<223>  Sac7d/Aho7c consensus sequence Albumin-binding protein B11

<220>
<221>  VARIANT
<222>  (1)..(1)
<223>  Xaa is V or T

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (36)..(36)
<223>  Xaa is N or Q

<220>
<221>  VARIANT
<222>  (55)..(64)
<223>  XaaXaaXaaXaaXaaXaaXaaXaaXaaXaa is DMLARAEREK or EKLK------

<400>  44

Xaa Lys Val Lys Phe Trp Pro Arg Gly Glu Glu Lys Val Val Xaa Xaa
1               5                   10                  15

Ser Lys Ile Ala Trp Val Leu Arg Ala Asp Lys Thr Xaa Met Phe Lys
            20                  25                  30

Tyr Asp Asp Xaa Gly Lys Lys Gly Tyr Gly Val Val Leu Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  45

<211>  64
<212>  PRT
<213>  Artificial Sequence

<220>
<223>  Sac7d-derived Albumin-binding protein


<220>
<221>  VARIANT
<222>  (6)..(6)
<223>  Xaa is W,F,Y,V,I or L

<220>
<221>  VARIANT
<222>  (7)..(7)
<223>  Xaa is P or F

<220>
<221>  VARIANT
<222>  (8)..(8)
<223>  Xaa is R,K,S,T,Q or N

<220>
<221>  VARIANT
<222>  (15)..(15)
<223>  Xaa is D or E

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (43)..(43)
<223>  Xaa is V or F

<220>
<221>  VARIANT
<222>  (45)..(45)
<223>  Xaa is any amino acid

<220>
<221>  VARIANT
<222>  (56)..(56)
<223>  Xaa is M or L

<400>  45

Val Lys Val Lys Phe Xaa Xaa Xaa Gly Glu Glu Lys Glu Val Xaa Thr
1               5                   10                  15

Ser Lys Ile Xaa Trp Val Leu Arg Xaa Gly Lys Xaa Val Xaa Phe Lys
            20                  25                  30

Tyr Asp Asp Xaa Gly Lys Xaa Gly Tyr Gly Xaa Val Xaa Glu Lys Asp
        35                  40                  45

Ala Pro Lys Glu Leu Leu Asp Xaa Leu Ala Arg Ala Glu Arg Glu Lys
    50                  55                  60

<210>  46
<211>  64
<212>  PRT
<213>  Artificial Sequence

<220>
<223>  Sac7d Albumin-binding protein F06

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (15)..(15)
<223>  Xaa is D or E

<220>
<221>  VARIANT
<222>  (20)..(20)
<223>  Xaa is any amino acid

<220>
<221>  VARIANT
<222>  (25)..(25)
<223>  Xaa is any amino acid

<220>

<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)
<223>  Xaa is any amino acid

<220>
<221>  VARIANT
<222>  (56)..(56)
<223>  Xaa is M or L

<400>  46

Val Lys Val Lys Phe Xaa Phe Xaa Gly Glu Glu Lys Glu Val Xaa Thr
1               5                   10                  15

Ser Lys Ile Xaa Trp Val Leu Arg Xaa Gly Lys Xaa Val Xaa Phe Lys
            20                  25                  30

Tyr Asp Asp Xaa Gly Lys Xaa Gly Tyr Gly Phe Val Xaa Glu Lys Asp
        35                  40                  45

Ala Pro Lys Glu Leu Leu Asp Xaa Leu Ala Arg Ala Glu Arg Glu Lys
    50                  55                  60

<210>  47
<211>  64
<212>  PRT
<213>  Artificial Sequence

<220>
<223>  Sac7d Albumin-binding protein F06

<220>
<221>  VARIANT
<222>  (15)..(15)
<223>  Xaa is D or E

<220>
<221>  VARIANT

<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)
<223>  Xaa is any amino acid

<220>
<221>  VARIANT
<222>  (56)..(56)
<223>  Xaa is M or L

<400>  47

Val Lys Val Lys Phe Val Phe Ser Gly Glu Glu Lys Glu Val Xaa Thr
1               5                   10                  15

Ser Lys Ile Xaa Trp Val Leu Arg Xaa Gly Lys Xaa Val Xaa Phe Lys
            20                  25                  30

Tyr Asp Asp Xaa Gly Lys Xaa Gly Tyr Gly Phe Val Xaa Glu Lys Asp
        35                  40                  45

Ala Pro Lys Glu Leu Leu Asp Xaa Leu Ala Arg Ala Glu Arg Glu Lys
    50                  55                  60

<210>  48
<211>  64
<212>  PRT
<213>  Artificial Sequence

<220>
<223>  Sac7d Albumin-binding protein F06

<220>
<221> VARIANT
<222> (15)..(15)
<223> Xaa is D or E

<220>
<221> VARIANT
<222> (29)..(29)
<223> Xaa is V or I

<220>
<221> misc_feature
<222> (36)..(36)
<223> Xaa can be any naturally occurring amino acid

<220>
<221> VARIANT
<222> (56)..(56)
<223> Xaa is M or L

<400> 48

Val Lys Val Lys Phe Val Phe Ser Gly Glu Glu Lys Glu Val Xaa Thr
1               5                   10                  15

Ser Lys Ile Lys Trp Val Leu Arg Trp Gly Lys Ala Val Gly Phe Lys
            20                  25                  30

Tyr Asp Asp Xaa Gly Lys Ile Gly Tyr Gly Phe Val Ala Glu Lys Asp
        35                  40                  45

Ala Pro Lys Glu Leu Leu Asp Xaa Leu Ala Arg Ala Glu Arg Glu Lys
    50                  55                  60

<210> 49
<211> 64
<212> PRT
<213> Artificial Sequence

<220>
<223> Sac7d Albumin-binding protein B11

<220>
<221> VARIANT
<222> (6)..(6)
<223> Xaa is W,F,Y,V,I or L

<220>
<221> VARIANT
<222> (8)..(8)
<223> Xaa is R,K,S,T,Q or N

<220>
<221> VARIANT
<222> (15)..(15)
<223> Xaa is D or E

<220>
<221> VARIANT
<222> (20)..(20)
<223> Xaa is any amino acid

<220>
<221> VARIANT
<222> (25)..(25)
<223> Xaa is any amino acid

<220>
<221> VARIANT
<222> (28)..(28)
<223> Xaa is any amino acid

<220>
<221> VARIANT
<222> (30)..(30)
<223> Xaa is any amino acid

<220>
<221> VARIANT
<222> (36)..(36)
<223> Xaa is N or Q

<220>
<221> VARIANT
<222> (39)..(39)
<223> Xaa is any amino acid

<220>
<221> VARIANT
<222> (45)..(45)
<223> Xaa is any amino acid

<220>
<221> VARIANT
<222> (56)..(56)
<223> Xaa is M or L

<400> 49

Val Lys Val Lys Phe Xaa Pro Xaa Gly Glu Glu Lys Glu Val Xaa Thr
1               5                   10                  15

Ser Lys Ile Xaa Trp Val Leu Arg Xaa Gly Lys Xaa Val Xaa Phe Lys
            20                  25                  30

Tyr Asp Asp Xaa Gly Lys Xaa Gly Tyr Gly Val Val Xaa Glu Lys Asp
        35                  40                  45

Ala Pro Lys Glu Leu Leu Asp Xaa Leu Ala Arg Ala Glu Arg Glu Lys
    50                  55                  60

<210> 50
<211> 64
<212> PRT
<213> Artificial Sequence

<220>
<223>  Sac7d Albumin-binding protein B11

<220>
<221>  VARIANT
<222>  (15)..(15)
<223>  Xaa is D or E

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)
<223>  Xaa is any amino acid

<220>
<221>  VARIANT
<222>  (56)..(56)
<223>  Xaa is M or L

<400>  50

Val Lys Val Lys Phe Trp Pro Arg Gly Glu Glu Lys Glu Val Xaa Thr
1               5                   10                  15

Ser Lys Ile Xaa Trp Val Leu Arg Xaa Gly Lys Xaa Val Xaa Phe Lys
            20                  25                  30

Tyr Asp Asp Xaa Gly Lys Xaa Gly Tyr Gly Val Val Xaa Glu Lys Asp
        35                  40                  45

Ala Pro Lys Glu Leu Leu Asp Xaa Leu Ala Arg Ala Glu Arg Glu Lys
    50                  55                  60

<210>  51
<211>  64
<212>  PRT
<213>  Artificial Sequence

<220>
<223>  Sac7d Albumin-binding protein B11

<220>
<221>  VARIANT
<222>  (15)..(15)
<223>  Xaa is D or E

<220>
<221>  VARIANT
<222>  (36)..(36)
<223>  Xaa is N or Q

<220>
<221>  VARIANT
<222>  (56)..(56)
<223>  Xaa is M or L

<400>  51

Val Lys Val Lys Phe Trp Pro Arg Gly Glu Glu Lys Val Val Xaa Thr
1               5                   10                  15

Ser Lys Ile Ala Trp Val Leu Arg Ala Asp Lys Thr Val Met Phe Lys
            20                  25                  30

Tyr Asp Asp Xaa Gly Lys Lys Gly Tyr Gly Val Val Leu Glu Lys Asp
        35                  40                  45

Ala Pro Lys Glu Leu Leu Asp Xaa Leu Ala Arg Ala Glu Arg Glu Lys
    50                  55                  60

<210>  52
<211>  64
<212>  PRT
<213>  Artificial Sequence

<220>
<223>  Sac7d-derived Albumin-binding protein

<220>
<221>  VARIANT
<222>  (6)..(6)
<223>  Xaa is W,F,Y,V,I or L

<220>
<221>  VARIANT

<222>  (7)..(7)
<223>  Xaa is P or F

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (43)..(43)
<223>  Xaa is V or F

<220>
<221>  VARIANT
<222>  (45)..(45)
<223>  Xaa is any amino acid

<400>  52

Val Lys Val Lys Phe Xaa Xaa Xaa Gly Glu Glu Lys Glu Val Asp Thr
1               5                   10                  15

Ser Lys Ile Xaa Trp Val Leu Arg Xaa Gly Lys Xaa Val Xaa Phe Lys
            20                  25                  30

Tyr Asp Asp Asn Gly Lys Xaa Gly Tyr Gly Xaa Val Xaa Glu Lys Asp
        35                  40                  45

Ala Pro Lys Glu Leu Leu Asp Met Leu Ala Arg Ala Glu Arg Glu Lys
    50                  55                  60

<210>  53
<211>  64

<212>  PRT
<213>  Artificial Sequence

<220>
<223>  Sac7d Albumin-binding protein F06



<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  53

Val Lys Val Lys Phe Xaa Phe Xaa Gly Glu Glu Lys Glu Val Asp Thr
1               5                   10                  15


Ser Lys Ile Xaa Trp Val Leu Arg Xaa Gly Lys Xaa Val Xaa Phe Lys
            20                  25                  30


Tyr Asp Asp Asn Gly Lys Xaa Gly Tyr Gly Phe Val Xaa Glu Lys Asp
        35                  40                  45


Ala Pro Lys Glu Leu Leu Asp Met Leu Ala Arg Ala Glu Arg Glu Lys
    50                  55                  60

<210>  54
<211>  64
<212>  PRT
<213>  Artificial Sequence

<220>
<223>  Sac7d Albumin-binding protein F06


<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  54

Val Lys Val Lys Phe Val Phe Ser Gly Glu Glu Lys Glu Val Asp Thr
1               5                   10                  15


Ser Lys Ile Xaa Trp Val Leu Arg Xaa Gly Lys Xaa Val Xaa Phe Lys
            20                  25                  30


Tyr Asp Asp Asn Gly Lys Xaa Gly Tyr Gly Phe Val Xaa Glu Lys Asp
        35                  40                  45


Ala Pro Lys Glu Leu Leu Asp Met Leu Ala Arg Ala Glu Arg Glu Lys
    50                  55                  60


<210>  55
<211>  64
<212>  PRT
<213>  Artificial Sequence

<220>
<223>  Sac7d Albumin-binding protein F06

<400>  55

Val Lys Val Lys Phe Val Phe Ser Gly Glu Glu Lys Glu Val Asp Thr
1               5                   10                  15

Ser Lys Ile Lys Trp Val Leu Arg Trp Gly Lys Ala Val Gly Phe Lys
            20                  25                  30

Tyr Asp Asp Asn Gly Lys Ile Gly Tyr Gly Phe Val Ala Glu Lys Asp
        35                  40                  45

Ala Pro Lys Glu Leu Leu Asp Met Leu Ala Arg Ala Glu Arg Glu Lys
    50                  55                  60

<210>  56
<211>  64
<212>  PRT
<213>  Artificial Sequence

<220>
<223>  Sac7d Albumin-binding protein B11

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT

<222>  (39)..(39)
<223>  Xaa is any amino acid

<220>
<221>  VARIANT
<222>  (45)..(45)
<223>  Xaa is any amino acid

<400>  56

Val Lys Val Lys Phe Xaa Pro Xaa Gly Glu Glu Lys Glu Val Asp Thr
1               5                   10                  15

Ser Lys Ile Xaa Trp Val Leu Arg Xaa Gly Lys Xaa Val Xaa Phe Lys
            20                  25                  30

Tyr Asp Asp Asn Gly Lys Xaa Gly Tyr Gly Val Val Xaa Glu Lys Asp
        35                  40                  45

Ala Pro Lys Glu Leu Leu Asp Met Leu Ala Arg Ala Glu Arg Glu Lys
    50                  55                  60

<210>  57
<211>  64
<212>  PRT
<213>  Artificial Sequence

<220>
<223>  Sac7d Albumin-binding protein B11

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)

<223>  Xaa is any amino acid

<400>  57

Val Lys Val Lys Phe Trp Pro Arg Gly Glu Glu Lys Glu Val Asp Thr
1               5                   10                  15

Ser Lys Ile Xaa Trp Val Leu Arg Xaa Gly Lys Xaa Val Xaa Phe Lys
            20                  25                  30

Tyr Asp Asp Asn Gly Lys Xaa Gly Tyr Gly Val Val Xaa Glu Lys Asp
        35                  40                  45

Ala Pro Lys Glu Leu Leu Asp Met Leu Ala Arg Ala Glu Arg Glu Lys
    50                  55                  60

<210>  58
<211>  64
<212>  PRT
<213>  Artificial Sequence

<220>
<223>  Sac7d Albumin-binding protein B11

<400>  58

Val Lys Val Lys Phe Trp Pro Arg Gly Glu Glu Lys Val Val Asp Thr
1               5                   10                  15

Ser Lys Ile Ala Trp Val Leu Arg Ala Asp Lys Thr Val Met Phe Lys
            20                  25                  30

Tyr Asp Asp Asn Gly Lys Lys Gly Tyr Gly Val Val Leu Glu Lys Asp
        35                  40                  45

Ala Pro Lys Glu Leu Leu Asp Met Leu Ala Arg Ala Glu Arg Glu Lys
    50                  55                  60

<210>  59
<211>  64
<212>  PRT
<213>  Artificial Sequence

<220>
<223>  Sac7d Albumin-binding protein

<220>
<221>  VARIANT
<222>  (6)..(6)
<223>  Xaa is W,F,Y,V,I or L

<220>
<221>  VARIANT
<222>  (7)..(7)

<223>  Xaa is P or F

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (43)..(43)
<223>  Xaa is V or F

<220>
<221>  VARIANT
<222>  (45)..(45)
<223>  Xaa is any amino acid

<400>  59

Val Lys Val Lys Phe Xaa Xaa Xaa Gly Glu Glu Lys Glu Val Glu Thr
1               5                   10                  15

Ser Lys Ile Xaa Trp Val Leu Arg Xaa Gly Lys Xaa Val Xaa Phe Lys
            20                  25                  30

Tyr Asp Asp Gln Gly Lys Xaa Gly Tyr Gly Xaa Val Xaa Glu Lys Asp
        35                  40                  45

Ala Pro Lys Glu Leu Leu Asp Leu Leu Ala Arg Ala Glu Arg Glu Lys
    50                  55                  60

<210>  60
<211>  64
<212>  PRT

<213>  Artificial Sequence

<220>
<223>  Sac7d Albumin-binding protein F06


<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  60

Val Lys Val Lys Phe Xaa Phe Xaa Gly Glu Glu Lys Glu Val Glu Thr
1               5                   10                  15


Ser Lys Ile Xaa Trp Val Leu Arg Xaa Gly Lys Xaa Val Xaa Phe Lys
            20                  25                  30


Tyr Asp Asp Gln Gly Lys Xaa Gly Tyr Gly Phe Val Xaa Glu Lys Asp
        35                  40                  45


Ala Pro Lys Glu Leu Leu Asp Leu Leu Ala Arg Ala Glu Arg Glu Lys
    50                  55                  60

<210>  61
<211>  64
<212>  PRT
<213>  Artificial Sequence

<220>
<223>  Sac7d Albumin-binding protein F06


<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  61

Val Lys Val Lys Phe Val Phe Ser Gly Glu Glu Lys Glu Val Glu Thr
1               5                   10                  15


Ser Lys Ile Xaa Trp Val Leu Arg Xaa Gly Lys Xaa Val Xaa Phe Lys
            20                  25                  30


Tyr Asp Asp Gln Gly Lys Xaa Gly Tyr Gly Phe Val Xaa Glu Lys Asp
        35                  40                  45


Ala Pro Lys Glu Leu Leu Asp Leu Leu Ala Arg Ala Glu Arg Glu Lys
    50                  55                  60


<210>  62
<211>  64
<212>  PRT
<213>  Artificial Sequence

<220>
<223>  Sac7d Albumin-binding protein F06

<400>  62

Val Lys Val Lys Phe Val Phe Ser Gly Glu Glu Lys Glu Val Glu Thr
1               5                   10                  15

Ser Lys Ile Lys Trp Val Leu Arg Trp Gly Lys Ala Val Gly Phe Lys
            20                  25                  30

Tyr Asp Asp Gln Gly Lys Ile Gly Tyr Gly Phe Val Ala Glu Lys Asp
        35                  40                  45

Ala Pro Lys Glu Leu Leu Asp Leu Leu Ala Arg Ala Glu Arg Glu Lys
    50                  55                  60

<210>  63
<211>  64
<212>  PRT
<213>  Artificial Sequence

<220>
<223>  Sac7d Albumin-binding protein B11

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)

<223>  Xaa is any amino acid

<220>
<221>  VARIANT
<222>  (45)..(45)
<223>  Xaa is any amino acid

<400>  63

Val Lys Val Lys Phe Xaa Pro Xaa Gly Glu Glu Lys Glu Val Glu Thr
1               5                   10                  15

Ser Lys Ile Xaa Trp Val Leu Arg Xaa Gly Lys Xaa Val Xaa Phe Lys
            20                  25                  30

Tyr Asp Asp Gln Gly Lys Xaa Gly Tyr Gly Val Val Xaa Glu Lys Asp
        35                  40                  45

Ala Pro Lys Glu Leu Leu Asp Leu Leu Ala Arg Ala Glu Arg Glu Lys
    50                  55                  60

<210>  64
<211>  64
<212>  PRT
<213>  Artificial Sequence

<220>
<223>  Sac7d Albumin-binding protein B11

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  64

Val Lys Val Lys Phe Trp Pro Arg Gly Glu Glu Lys Glu Val Glu Thr
1               5                   10                  15

Ser Lys Ile Xaa Trp Val Leu Arg Xaa Gly Lys Xaa Val Xaa Phe Lys
            20                  25                  30

Tyr Asp Asp Gln Gly Lys Xaa Gly Tyr Gly Val Val Xaa Glu Lys Asp
        35                  40                  45

Ala Pro Lys Glu Leu Leu Asp Leu Leu Ala Arg Ala Glu Arg Glu Lys
    50                  55                  60

<210>  65
<211>  64
<212>  PRT
<213>  Artificial Sequence

<220>
<223>  Sac7d Albumin-binding protein B11

<400>  65

Val Lys Val Lys Phe Trp Pro Arg Gly Glu Glu Lys Val Val Glu Thr
1               5                   10                  15

Ser Lys Ile Ala Trp Val Leu Arg Ala Asp Lys Thr Val Met Phe Lys
            20                  25                  30

Tyr Asp Asp Gln Gly Lys Lys Gly Tyr Gly Val Val Leu Glu Lys Asp
        35                  40                  45

Ala Pro Lys Glu Leu Leu Asp Leu Leu Ala Arg Ala Glu Arg Glu Lys
    50                  55                  60

<210>  66
<211>  58
<212>  PRT
<213>  Artificial Sequence

<220>
<223>  Aho7c sequence Albumin-binding protein

<220>
<221>  VARIANT
<222>  (6)..(6)
<223>  Xaa is W,F,Y,V,I or L

<220>
<221>  VARIANT
<222>  (7)..(7)
<223>  Xaa is P or F

<220>
<221>  VARIANT
<222>  (8)..(8)
<223>  Xaa is R,K,S,T,Q or N

<220>
<221>  VARIANT
<222>  (15)..(15)
<223>  Xaa is D or E

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (43)..(43)
<223>  Xaa is V or F

<220>
<221>  VARIANT
<222>  (45)..(45)
<223>  Xaa is any amino acid

<400>  66

Thr Lys Val Lys Phe Xaa Xaa Xaa Gly Glu Glu Lys Glu Val Xaa Ile
1               5                   10                  15

Ser Lys Ile Xaa Trp Val Leu Arg Xaa Gly Lys Xaa Ile Xaa Phe Lys
            20                  25                  30

Tyr Asp Asp Xaa Gly Lys Xaa Gly Tyr Gly Xaa Val Xaa Glu Lys Asp
        35                  40                  45

Ala Pro Lys Glu Leu Leu Glu Lys Leu Lys
    50                  55

<210>  67
<211>  58
<212>  PRT
<213>  Artificial Sequence

<220>
<223>  Aho7c sequence Albumin-binding protein F06

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (15)..(15)
<223>  Xaa is D or E

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)

<223>  Xaa is any amino acid

<400>  67

Thr Lys Val Lys Phe Xaa Phe Xaa Gly Glu Glu Lys Glu Val Xaa Ile
1               5                   10                  15

Ser Lys Ile Xaa Trp Val Leu Arg Xaa Gly Lys Xaa Ile Xaa Phe Lys
            20                  25                  30

Tyr Asp Asp Xaa Gly Lys Xaa Gly Tyr Gly Phe Val Xaa Glu Lys Asp
        35                  40                  45

Ala Pro Lys Glu Leu Leu Glu Lys Leu Lys
    50                  55

<210>  68
<211>  58
<212>  PRT
<213>  Artificial Sequence

<220>
<223>  Aho7c sequence Albumin-binding protein F06

<220>
<221>  VARIANT
<222>  (15)..(15)
<223>  Xaa is D or E

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)
<223>  Xaa is any amino acid

<400>  68

Thr Lys Val Lys Phe Val Phe Ser Gly Glu Glu Lys Glu Val Xaa Ile
1               5                   10                  15

Ser Lys Ile Xaa Trp Val Leu Arg Xaa Gly Lys Xaa Ile Xaa Phe Lys
            20                  25                  30

Tyr Asp Asp Xaa Gly Lys Xaa Gly Tyr Gly Phe Val Xaa Glu Lys Asp
        35                  40                  45

Ala Pro Lys Glu Leu Leu Glu Lys Leu Lys
    50                  55

<210>  69
<211>  58
<212>  PRT
<213>  Artificial Sequence

<220>
<223>  Aho7c sequence Albumin-binding protein F06

<220>
<221>  VARIANT
<222>  (15)..(15)
<223>  Xaa is D or E

<220>
<221>  VARIANT
<222>  (29)..(29)
<223>  Xaa is V or I

<220>
<221>  misc_feature
<222>  (36)..(36)
<223>  Xaa can be any naturally occurring amino acid

<400>  69

Thr Lys Val Lys Phe Val Phe Ser Gly Glu Glu Lys Glu Val Xaa Ile
1               5                   10                  15

Ser Lys Ile Lys Trp Val Leu Arg Trp Gly Lys Ala Ile Gly Phe Lys
            20                  25                  30

Tyr Asp Asp Xaa Gly Lys Ile Gly Tyr Gly Phe Val Ala Glu Lys Asp
        35                  40                  45

Ala Pro Lys Glu Leu Leu Glu Lys Leu Lys

50                  55

<210>  70
<211>  58
<212>  PRT
<213>  Artificial Sequence

<220>
<223>  Aho7c sequence Albumin-binding protein B11


<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (15)..(15)
<223>  Xaa is D or E

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (45)..(45)
<223>  Xaa is any amino acid

<400>  70

Thr Lys Val Lys Phe Xaa Pro Xaa Gly Glu Glu Lys Glu Val Xaa Ile
1               5                   10                  15

Ser Lys Ile Xaa Trp Val Leu Arg Xaa Gly Lys Xaa Ile Xaa Phe Lys
            20                  25                  30

Tyr Asp Asp Xaa Gly Lys Xaa Gly Tyr Gly Val Val Xaa Glu Lys Asp
        35                  40                  45

Ala Pro Lys Glu Leu Leu Glu Lys Leu Lys
    50                  55

<210>  71
<211>  58
<212>  PRT
<213>  Artificial Sequence

<220>
<223>  Aho7c sequence Albumin-binding protein B11

<220>
<221>  VARIANT
<222>  (15)..(15)
<223>  Xaa is D or E

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT

<222>  (45)..(45)
<223>  Xaa is any amino acid

<400>  71

Thr Lys Val Lys Phe Trp Pro Arg Gly Glu Glu Lys Glu Val Xaa Ile
1               5                   10                  15

Ser Lys Ile Xaa Trp Val Leu Arg Xaa Gly Lys Xaa Ile Xaa Phe Lys
            20                  25                  30

Tyr Asp Asp Xaa Gly Lys Xaa Gly Tyr Gly Val Val Xaa Glu Lys Asp
        35                  40                  45

Ala Pro Lys Glu Leu Leu Glu Lys Leu Lys
    50                  55

<210>  72
<211>  58
<212>  PRT
<213>  Artificial Sequence

<220>
<223>  Aho7c sequence Albumin-binding protein B11

<220>
<221>  VARIANT
<222>  (15)..(15)
<223>  Xaa is D or E

<220>
<221>  VARIANT
<222>  (36)..(36)
<223>  Xaa is N or Q

<400>  72

Thr Lys Val Lys Phe Trp Pro Arg Gly Glu Glu Lys Val Val Xaa Ile
1               5                   10                  15

Ser Lys Ile Ala Trp Val Leu Arg Ala Asp Lys Thr Ile Met Phe Lys
            20                  25                  30

Tyr Asp Asp Xaa Gly Lys Lys Gly Tyr Gly Val Val Leu Glu Lys Asp
        35                  40                  45

Ala Pro Lys Glu Leu Leu Glu Lys Leu Lys
    50                  55

<210>  73
<211>  58
<212>  PRT
<213>  Artificial Sequence

<220>
<223>  Aho7c sequence Albumin-binding protein



<220>
<221>  VARIANT
<222>  (6)..(6)
<223>  Xaa is W,F,Y,V,I or L

<220>
<221>  VARIANT
<222>  (7)..(7)
<223>  Xaa is P or F

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (43)..(43)
<223>  Xaa is V or F

<220>
<221>  VARIANT
<222>  (45)..(45)
<223>  Xaa is any amino acid

<400>  73

Thr Lys Val Lys Phe Xaa Xaa Xaa Gly Glu Glu Lys Glu Val Asp Ile
1               5                   10                  15

Ser Lys Ile Xaa Trp Val Leu Arg Xaa Gly Lys Xaa Ile Xaa Phe Lys
            20                  25                  30

Tyr Asp Asp Asn Gly Lys Xaa Gly Tyr Gly Xaa Val Xaa Glu Lys Asp
        35                  40                  45

Ala Pro Lys Glu Leu Leu Glu Lys Leu Lys
    50                  55

<210>  74
<211>  58
<212>  PRT
<213>  Artificial Sequence

<220>
<223>  Aho7c sequence Albumin-binding protein F06

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  74

Thr Lys Val Lys Phe Xaa Phe Xaa Gly Glu Glu Lys Glu Val Asp Ile
1               5                   10                  15

Ser Lys Ile Xaa Trp Val Leu Arg Xaa Gly Lys Xaa Ile Xaa Phe Lys
            20                  25                  30

Tyr Asp Asp Asn Gly Lys Xaa Gly Tyr Gly Phe Val Xaa Glu Lys Asp
        35                  40                  45

Ala Pro Lys Glu Leu Leu Glu Lys Leu Lys
    50                  55

<210>  75
<211>  58
<212>  PRT
<213>  Artificial Sequence

<220>
<223>  Aho7c sequence Albumin-binding protein F06

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  75

Thr Lys Val Lys Phe Val Phe Ser Gly Glu Glu Lys Glu Val Asp Ile
1               5                   10                  15

Ser Lys Ile Xaa Trp Val Leu Arg Xaa Gly Lys Xaa Ile Xaa Phe Lys
            20                  25                  30

Tyr Asp Asp Asn Gly Lys Xaa Gly Tyr Gly Phe Val Xaa Glu Lys Asp
        35                  40                  45

Ala Pro Lys Glu Leu Leu Glu Lys Leu Lys
    50                  55

<210>  76
<211>  58
<212>  PRT
<213>  Artificial Sequence

<220>
<223>  Aho7c sequence Albumin-binding protein F06

<400>  76

Thr Lys Val Lys Phe Val Phe Ser Gly Glu Glu Lys Glu Val Asp Ile
1               5                   10                  15

Ser Lys Ile Lys Trp Val Leu Arg Trp Gly Lys Ala Ile Gly Phe Lys
            20                  25                  30

Tyr Asp Asp Asn Gly Lys Ile Gly Tyr Gly Phe Val Ala Glu Lys Asp
        35                  40                  45

Ala Pro Lys Glu Leu Leu Glu Lys Leu Lys
    50                  55

<210>  77
<211>  58
<212>  PRT
<213>  Artificial Sequence

<220>
<223>  Aho7c sequence Albumin-binding protein B11

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>

<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  77

Thr Lys Val Lys Phe Xaa Pro Xaa Gly Glu Glu Lys Glu Val Asp Ile
1               5                   10                  15

Ser Lys Ile Xaa Trp Val Leu Arg Xaa Gly Lys Xaa Ile Xaa Phe Lys
            20                  25                  30

Tyr Asp Asp Asn Gly Lys Xaa Gly Tyr Gly Val Val Xaa Glu Lys Asp
        35                  40                  45

Ala Pro Lys Glu Leu Leu Glu Lys Leu Lys
    50                  55

<210>  78
<211>  58
<212>  PRT
<213>  Artificial Sequence

<220>
<223>  Aho7c sequence Albumin-binding protein B11

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT

<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  78

Thr Lys Val Lys Phe Trp Pro Arg Gly Glu Glu Lys Glu Val Asp Ile
1               5                   10                  15

Ser Lys Ile Xaa Trp Val Leu Arg Xaa Gly Lys Xaa Ile Xaa Phe Lys
            20                  25                  30

Tyr Asp Asp Asn Gly Lys Xaa Gly Tyr Gly Val Val Xaa Glu Lys Asp
        35                  40                  45

Ala Pro Lys Glu Leu Leu Glu Lys Leu Lys
    50                  55

<210>  79
<211>  58
<212>  PRT
<213>  Artificial Sequence

<220>
<223>  Aho7c sequence Albumin-binding protein B11

<220>
<221>  misc_feature
<222>  (15)..(15)
<223>  Xaa can be any naturally occurring amino acid

<220>
<221>  misc_feature
<222>  (36)..(36)
<223>  Xaa can be any naturally occurring amino acid

<400>  79

Thr Lys Val Lys Phe Trp Pro Arg Gly Glu Glu Lys Val Val Xaa Ile
1               5                   10                  15

Ser Lys Ile Ala Trp Val Leu Arg Ala Asp Lys Thr Ile Met Phe Lys
            20                  25                  30

Tyr Asp Asp Xaa Gly Lys Lys Gly Tyr Gly Val Val Leu Glu Lys Asp
        35                  40                  45

Ala Pro Lys Glu Leu Leu Glu Lys Leu Lys
    50                  55

<210>  80
<211>  58
<212>  PRT
<213>  Artificial Sequence

<220>
<223>  Aho7c sequence Albumin-binding protein

<220>
<221>  VARIANT
<222>  (6)..(6)
<223>  Xaa is W,F,Y,V,I or L

<220>
<221>  VARIANT
<222>  (7)..(7)
<223>  Xaa is P or F

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (36)..(36)
<223>  Xaa is N or Q

<220>
<221>  VARIANT
<222>  (39)..(39)
<223>  Xaa is any amino acid

<220>
<221>  VARIANT
<222>  (43)..(43)
<223>  Xaa is V or F

<220>
<221>  VARIANT
<222>  (45)..(45)
<223>  Xaa is any amino acid

<400>  80

Thr Lys Val Lys Phe Xaa Xaa Xaa Gly Glu Glu Lys Glu Val Glu Ile
1               5                   10                  15

Ser Lys Ile Xaa Trp Val Leu Arg Xaa Gly Lys Xaa Ile Xaa Phe Lys
            20                  25                  30

Tyr Asp Asp Gln Gly Lys Xaa Gly Tyr Gly Xaa Val Xaa Glu Lys Asp
        35                  40                  45

Ala Pro Lys Glu Leu Leu Glu Lys Leu Lys
    50                  55

<210>  81
<211>  58
<212>  PRT
<213>  Artificial Sequence

<220>
<223>  Aho7c sequence Albumin-binding protein F06

<220>
<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  81

Thr Lys Val Lys Phe Xaa Phe Xaa Gly Glu Glu Lys Glu Val Glu Ile
1               5                   10                  15

Ser Lys Ile Xaa Trp Val Leu Arg Xaa Gly Lys Xaa Ile Xaa Phe Lys
            20                  25                  30

Tyr Asp Asp Gln Gly Lys Xaa Gly Tyr Gly Phe Val Xaa Glu Lys Asp
        35                  40                  45

Ala Pro Lys Glu Leu Leu Glu Lys Leu Lys
    50                  55

<210>  82
<211>  58
<212>  PRT
<213>  Artificial Sequence

<220>
<223>  Aho7c sequence Albumin-binding protein F06

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>

<221>  VARIANT
<222>  (39)..(39)
<223>  Xaa is any amino acid

<220>
<221>  VARIANT
<222>  (45)..(45)
<223>  Xaa is any amino acid

<400>  82

Thr Lys Val Lys Phe Val Phe Ser Gly Glu Glu Lys Glu Val Glu Ile
1               5                   10                  15

Ser Lys Ile Xaa Trp Val Leu Arg Xaa Gly Lys Xaa Ile Xaa Phe Lys
            20                  25                  30

Tyr Asp Asp Gln Gly Lys Xaa Gly Tyr Gly Phe Val Xaa Glu Lys Asp
        35                  40                  45

Ala Pro Lys Glu Leu Leu Glu Lys Leu Lys
    50                  55

<210>  83
<211>  58
<212>  PRT
<213>  Artificial Sequence

<220>
<223>  Aho7c sequence Albumin-binding protein F06

<400>  83

Thr Lys Val Lys Phe Val Phe Ser Gly Glu Glu Lys Glu Val Glu Ile
1               5                   10                  15

Ser Lys Ile Lys Trp Val Leu Arg Trp Gly Lys Ala Ile Gly Phe Lys
            20                  25                  30

Tyr Asp Asp Gln Gly Lys Ile Gly Tyr Gly Phe Val Ala Glu Lys Asp
        35                  40                  45

Ala Pro Lys Glu Leu Leu Glu Lys Leu Lys
    50                  55

<210>  84
<211>  58
<212>  PRT
<213>  Artificial Sequence

<220>
<223>  Aho7c sequence Albumin-binding protein B11

<220>

<221>  VARIANT
<222>  (6)..(6)
<223>  Xaa is W,F,Y,V,I or L

<220>
<221>  VARIANT
<222>  (8)..(8)
<223>  Xaa is R,K,S,T,Q or N

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  84

Thr Lys Val Lys Phe Xaa Pro Xaa Gly Glu Glu Lys Glu Val Glu Thr
1               5                   10                  15

Ser Lys Ile Xaa Trp Val Leu Arg Xaa Gly Lys Xaa Val Xaa Phe Lys
            20                  25                  30

Tyr Asp Asp Gln Gly Lys Xaa Gly Tyr Gly Val Val Xaa Glu Lys Asp
        35                  40                  45

Ala Pro Lys Glu Leu Leu Glu Lys Leu Lys
    50                  55

<210>  85
<211>  58
<212>  PRT
<213>  Artificial Sequence

<220>

<223>  Aho7c sequence Albumin-binding protein B11

<220>
<221>  VARIANT
<222>  (20)..(20)
<223>  Xaa is any amino acid

<220>
<221>  VARIANT
<222>  (25)..(25)
<223>  Xaa is any amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (39)..(39)
<223>  Xaa is any amino acid

<220>
<221>  VARIANT
<222>  (45)..(45)
<223>  Xaa is any amino acid

<400>  85

Thr Lys Val Lys Phe Trp Pro Arg Gly Glu Glu Lys Glu Val Glu Thr
1               5                   10                  15

Ser Lys Ile Xaa Trp Val Leu Arg Xaa Gly Lys Xaa Val Xaa Phe Lys
            20                  25                  30

Tyr Asp Asp Gln Gly Lys Xaa Gly Tyr Gly Val Val Xaa Glu Lys Asp
        35                  40                  45

Ala Pro Lys Glu Leu Leu Glu Lys Leu Lys
    50                  55

<210>  86
<211>  58
<212>  PRT
<213>  Artificial Sequence

<220>
<223>  Aho7c sequence Albumin-binding protein B11

<400>  86

Thr Lys Val Lys Phe Trp Pro Arg Gly Glu Glu Lys Val Val Glu Thr
1               5                   10                  15

Ser Lys Ile Ala Trp Val Leu Arg Ala Asp Lys Thr Val Met Phe Lys
            20                  25                  30

Tyr Asp Asp Gln Gly Lys Lys Gly Tyr Gly Val Val Leu Glu Lys Asp
        35                  40                  45

Ala Pro Lys Glu Leu Leu Glu Lys Leu Lys
    50                  55

<210>  87
<211>  64
<212>  PRT
<213>  Artificial Sequence

<220>
<223>  Consensus sequence Sac7d / Hso7c

<220>
<221>  VARIANT
<222>  (1)..(1)
<223>  Xaa is V or T

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (55)..(64)
<223>  Xaa is DMLARAEREK or EKLK------

<400>  87

Xaa Lys Val Lys Phe Lys Tyr Lys Gly Glu Glu Lys Glu Val Asp Xaa
1               5                   10                  15

Ser Lys Ile Lys Lys Val Trp Arg Val Gly Lys Met Xaa Ser Phe Thr
            20                  25                  30

Tyr Asp Asp Asn Gly Lys Thr Gly Arg Gly Ala Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60